# EUROPEAN PATENT SPECIFICATION

(11) **EP 4 618 006 B1**
(45) Date of publication and mention of the grant of the patent: **30.09.2026**
(21) Application number: 24162978.1
(22) Date of filing: 12.03.2024
(51) Int. Cl.: G06T 7/00, G06T 7/20

(54) **PROCESSING TECHNIQUES FOR OPTORETINOGRAPHY**
VERARBEITUNGSTECHNIKEN FÜR DIE OPTORETINOGRAPHIE
TECHNIQUES DE TRAITEMENT POUR L'OPTORÉTINOGRAPHIE

(43) Date of publication of application: 17.09.2025
(73) Proprietor: Optos plc, Dunfermline, Scotland KY11 8GR (GB)
(72) Inventor: PRECIADO, Miguel, Dunfermline, Scotland, KY11 8GR (GB); RYCROFT, Ewan, Dunfermline, Scotland, KY11 8GR (GB); McCOOL, Paul, Dunfermline, Scotland, KY11 8GR (GB); NORMAND, Margaret, Dunfermline, Scotland, KY11 8GR (GB)
(74) Representative: Hoffmann Eitle

(56) References cited:
- VIENOLA KARI V. ET AL: "Velocity-based optoretinography for clinical applications", OPTICA, vol. 9, no. 10, 22 September 2022 (2022-09-22), US, pages 1100 - 1108, XP093113345, ISSN: 2334-2536, [retrieved on 20220922], DOI: 10.1364/OPTICA.460835
- TAN BINGYAO ET AL: "Light-evoked deformations in rod photoreceptors, pigment epithelium and subretinal space revealed by prolonged and multilayered optoretinography", BIORXIV, 9 November 2023 (2023-11-09), pages 1 - 9, XP093194837, Retrieved from the Internet <URL:https://www.biorxiv.org/content/10.1101/2023.03.03.530926v2.full.pdf> [retrieved on 20231109], DOI: 10.1101/2023.03.03.530926
- MAKITA S ET AL: "Optical coherence angiography", OPTICS EXPRESS,, vol. 14, no. 17, 21 August 2006 (2006-08-21), pages 7821 - 7840, XP007903893, DOI: 10.1364/OE.14.007821

## Description

### [Field]

Example aspects herein generally relate to the field of optical coherence tomography (OCT) and, in particular, to techniques for processing OCT data generated by Fourier-domain OCT imaging systems to generate optoretinography (ORG) data indicative of a physiological response of a retina of an eye of a subject to an optical stimulus.

### [Background]

Optical coherence tomography (OCT) is an imaging technique based on low-coherence interferometry, which is widely used to acquire high-resolution two- and three-dimensional images of optical scattering media, such as biological tissue.

OCT imaging systems can be classified as being time-domain OCT (TD-OCT) or Fourier-domain OCT (FD-OCT) (also referred to as frequency-domain OCT), depending on how depth ranging is achieved. In TD-OCT, an optical path length of a reference arm of the imaging system's interferometer is varied in time during the acquisition of a reflectivity profile of the scattering medium being imaged by the OCT imaging system (referred to herein as the "imaging target"), the reflectivity profile being commonly referred to as a "depth scan" or "axial scan" ("A-scan"). In FD-OCT, a spectral interferogram resulting from an interference between light in the reference arm and light in the sample arm of the interferometer at each A-scan location is Fourier transformed to simultaneously acquire all points along the depth of the A-scan, without requiring any variation in the optical path length of the reference arm. FD-OCT can allow much faster imaging than scanning of the sample arm mirror in the interferometer, as all the back-reflections from the sample are measured simultaneously. Two common types of FD-OCT are spectral-domain OCT (SD-OCT) and swept-source OCT (SS-OCT). In SD-OCT, a broadband light source delivers many wavelengths to the imaging target, and all wavelengths are measured simultaneously using a spectrometer as the detector. In SS-OCT (also referred to as time-encoded frequency-domain OCT), the light source is swept through a range of wavelengths, and the temporal output of the detector is converted to spectral interference.

OCT imaging systems can also be classified as being point-scan (also known as "point detection" or "scanning point"), line-scan or full-field, depending on how the imaging system is configured to acquire OCT data at locations on the imaging target. A point-scan OCT imaging system acquires OCT data by scanning a focused sample beam across the surface of the imaging target, typically along a single line (which may be straight, or alternatively curved so as to define a circle or a spiral, for example) or along a set of (usually substantially parallel) lines on the surface of the imaging target, and acquiring an axial depth profile (A-scan) for each of a plurality of points along the line(s), one single point at a time, to build up OCT data comprising a one- or two-dimensional array of A-scans representing a two-dimensional (i.e. a B-scan) or three-dimensional (i.e. a C-scan or volumetric scan) reflectance profile of the sample.

A line-scan OCT imaging system acquires OCT data by scanning a focused line of light across the surface of the imaging target. Measured reflectance from the imaging target is used to generate OCT data comprising a two-dimensional reflectance profile (i.e. a B-scan) of the sample. By scanning the focused line of light across a plurality of locations on the imaging target, OCT data comprising a three-dimensional reflectance profile (i.e. a C-scan or volumetric scan) of the sample can be obtained. Typically, the focused line of light is straight and is scanned in a direction perpendicular to it, although in some instances it may be curved with the scanning direction adjusted accordingly. A full-field OCT imaging system acquires OCT data by projecting a beam of light onto the imaging target to acquire OCT data comprising a three-dimensional reflectance profile (i.e. a C-scan or volumetric scan) of the sample.

OCT imaging systems can also be classified as being phase-resolved, where both the intensity and phase of the light reflected from the imaging target are measured as a function of axial depth. Modern FD-OCT imaging systems often have a degree of phase stability that allows them to function as phase-resolved OCT imaging systems.

Optoretinography (ORG) generally refers to the detection of a physiological response of a retina of an eye to an optical stimulus (i.e. light-induced functional activity of the retina). ORG techniques include the non-invasive optical imaging of this physiological response of the retina. For example, OCT imaging systems can be used to image retinal neurons exhibiting a change in dimension (size) in response to excitation by the optical stimulus. These changes in dimension (typically changes in length of the photoreceptor outer segment (OS) in the retina, which is the difference in depth of the inner-outer segment (IS/OS) junction and the cone outer segment tip (COST) of the cone photoreceptors) lead to changes in the phase of the light waves returned from the eye that are big enough to be detectable by phase-resolved OCT imaging systems. The phase is very sensitive to movement in the tissue, and many phase-resolved OCT imaging systems are able to resolve displacements smaller than 10 nm, which may be much smaller than the axial resolution of the system or the wavelength of the light used in imaging.

Further background is provided in the following documents.

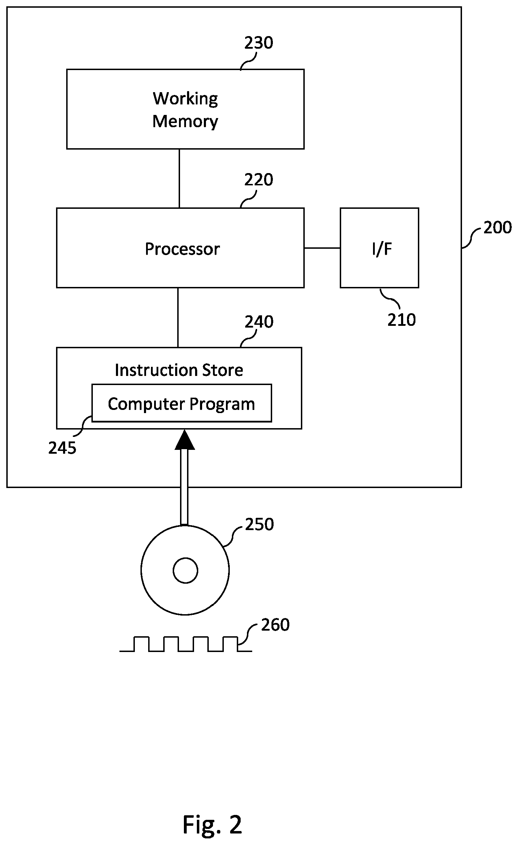

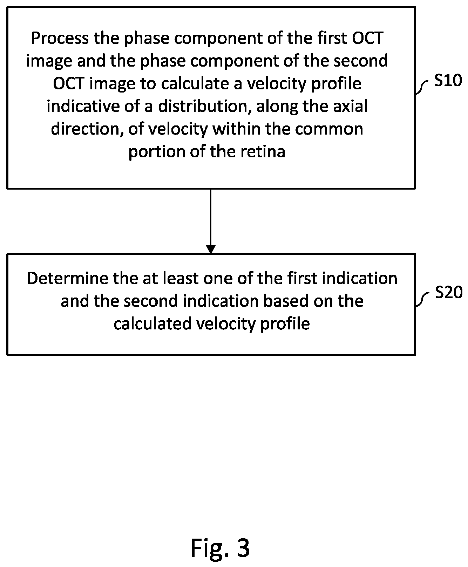

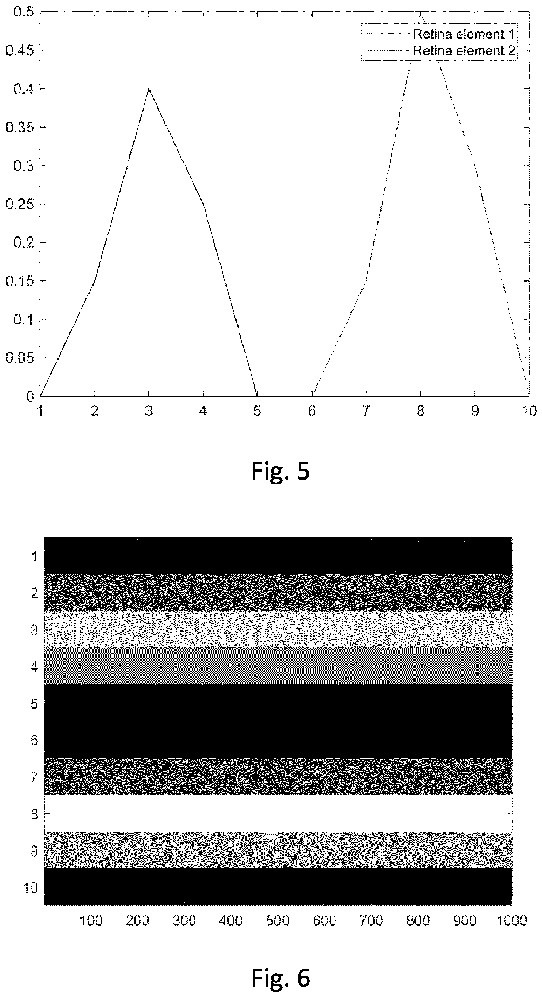

The article titled "Velocity-based optoretinography for clinical applications" by K. V. Vienola et al., published in Optica Vol. 9, No. 10, pages 1100-1108 (October 2022), describes measurements of the velocities of stimulus-evoked movements of subcellular features in the retina, using the phase information of reflected light to monitor the features' positions. This velocity-based approach was used measure the photoreceptor ORG responses in three healthy subjects. The measurements were made using the swept-source OCT (SS-OCT) system shown in Fig. 2 of the article, which was configured to scan in one dimension only, acquiring a series of B-scans in a single location. In Figure 3(b) of the article, the OCT amplitude is plotted as a function of depth, where the peaks originating from the IS/OS and COST are visible. In Fig. 3(a) of the article, the pseudocolour overlay represents the tissue velocity derived from phase slopes. Figure 6 of the article shows the outer segment velocity numerically integrated to reconstruct the change in OS length.

The article titled "Light-evoked deformations in rod photoreceptors, pigment epithelium and subretinal space revealed by prolonged and multilayered optoretinography" by B. Tan et al., pages 1-9, published on bioRxiv on 9 November 2023, discloses the use of a subpixel bulk motion correction algorithm, which enabled imaging of the nanometer-scale tissue dynamics during minute-long recordings, and unsupervised learning of spatiotemporal patterns, to discover optical signatures of the response to visual stimuli of retinal structures other than cones. These include inner and outer segments of rod photoreceptors, retinal pigment epithelium, and subretinal space in general. High sensitivity of the described technique enabled detection of the retinal responses to very dim stimuli: down to 0.01% bleach level, corresponding to natural levels of scotopic illumination. It was also demonstrated that with a single flash, the optoretinogram can map retinal responses across a 12° field of view, potentially replacing multifocal electroretinography, with its long acquisition time and low spatial resolution.

The article titled "Optical coherence angiography" by S. Makita et al., published in Optics Express, Vol. 14, No. 17, pages 7821-7840 (21 August 2006), discloses non-invasive angiography for the in vivo human eye. Three-dimensional flow imaging has been performed with high-speed spectral-domain optical coherence tomography. Sample motion is compensated by two algorithms. Axial motion between adjacent A-lines within one OCT image is compensated by the Doppler shift due to bulk sample motion. Axial displacements between neighbouring images are compensated by a correlation-based algorithm. Three-dimensional vasculature of ocular vessels has been visualized. By integrating volume sets of flow images, two-dimensional images of blood vessels are obtained. Retinal and choroidal blood vessel images are simultaneously obtained by separating the volume set into retinal part and choroidal parts. These are corresponding to fluorescein angiogram and indocyanine angiogram.

### [Summary]

The present invention provides a computer-implemented method according to claim 1, a computer program according to claim 17, and a data processing apparatus according to claim 18. Optional features are set out in the remaining claims.

There is provided, in accordance with a first example aspect herein, a computer-implemented method of processing a phase component (information) of a first OCT image and a phase component of a second OCT image of a sequence of OCT images of a common portion of a retina of an eye acquired by a Fourier-domain optical coherence tomography (OCT) imaging system after stimulation of the common portion by an optical stimulus, wherein the common portion comprises a layer of the retina (such as the outer segment (OS) of photoreceptor cells in the retina) whose thickness changed in response to the optical stimulus during acquisition of the sequence of OCT images, to determine at least one of a first indication of a first position along an axial (z-axis) direction in the OCT images of a first boundary of the layer and a second indication of a second position along the axial direction in the OCT images of a second, different boundary of the layer. The method comprises: processing the phase component of the first OCT image and the phase component of the second OCT image to calculate a velocity profile indicative of a distribution, along the axial direction, of velocity within the common portion of the retina; and determining the at least one of the first indication and the second indication based on the calculated velocity profile. The at least one of the first indication and the second indication is determined based on the calculated velocity profile by: determining, where the first indication is determined, a first position in the velocity profile corresponding to a maximum of velocity indicated by the velocity profile as the first indication; and determining, where the second indication is determined, a second position in the velocity profile corresponding to a minimum of velocity indicated by the velocity profile as the second indication. Thus, the velocity in a portion of the velocity profile corresponding to the layer in the retina may vary from a maximum velocity at a first position in the velocity profile corresponding to the first boundary of the layer at a location on the retina to a minimum velocity at a second position in the velocity profile corresponding to the second boundary of the layer at the location on the retina, and the first indication may indicate the first position in the velocity profile of the maximum velocity, and the second indication may indicate the second position in the velocity profile of the minimum velocity.

In some example embodiments, the at least one of the first indication and the second indication may be determined by processing the calculated velocity profile using a cluster analysis algorithm or a dimensionality reduction (e.g. component analysis) algorithm. The dimensionality reduction algorithm may comprise one of a principal component analysis (PCA) algorithm, an independent component analysis (ICA) algorithm, a linear discriminant analysis (LDA) algorithm or a non-negative matrix factorization (NMF) algorithm, for example. Where the dimensionality reduction algorithm comprises a PCA algorithm, the at least one of the first indication and the second indication may be determined using principal components that have been determined by applying the PCA algorithm to the calculated velocity profile.

Both the first position in the velocity profile and the second position in the velocity profile may be determined by: calculating, for each combination of a candidate first position in the velocity profile and a candidate second position in the velocity profile of all combinations of candidate first positions and candidate second positions in the velocity profile, a respective value of a difference between a respective velocity indicated by the velocity profile for the candidate first position and a respective velocity indicated by the velocity profile for the candidate second position in the combination; and identifying, as the first position in the velocity profile and the second position in the velocity profile, a candidate first position and a candidate second position of a combination in the plurality of combinations for which the calculated value of the difference is greatest among the calculated values of the difference.

Alternatively, both the first position in the velocity profile and the second position in the velocity profile are determined by: calculating, for each combination of a candidate first position in the velocity profile and a candidate second position in the velocity profile of all combinations of candidate first positions and candidate second positions in the velocity profile, a respective value of a difference between an average of respective velocities indicated by the velocity profile for a set of adjacent positions including the candidate first position in the combination, and an average of respective velocities indicated by the velocity profile for a set of adjacent positions including the candidate second position in the combination; and identifying, as the first position in the velocity profile and the second position in the velocity profile, the candidate first position and the candidate second position of a combination of the plurality of combinations for which the calculated value of the difference is greatest among the calculated values of the difference.

The computer-implemented method of the first example aspect or any of its example implementations set out above may further comprise one of: determining the first indication of the first position along the axial direction in the OCT images of the first boundary of the layer based on an amplitude component of at least one of the OCT images in the sequence of OCT images, wherein the second indication is determined by identifying the second position in the velocity profile corresponding to a minimum of velocity indicated by the velocity profile; and determining the second indication of the second position along the axial direction in the OCT images of the second boundary of the layer based on an amplitude component of at least one of the OCT images in the sequence of OCT images, wherein the first indication is determined by identifying the first position in the velocity profile corresponding to a maximum of velocity indicated by the velocity profile.

In some example embodiments, the computer-implemented method may further comprise calculating a comparison value being either (i) a value of an image quality metric calculated based on at least one of an amplitude component of the first OCT image and an amplitude component of the second OCT image or (ii) a value of an image similarity metric that provides a measure of a degree of similarity between images, the value of the image similarity metric being calculated based on the first OCT image and the second OCT image. The computer-implemented method may further comprise comparing the comparison value with a threshold to determine whether the comparison value is equal to or greater than the threshold. In a case where the comparison value is determined to be equal to or greater than the threshold, a first indicator, which indicates that the determined the at least one of the first indication and the second indication is reliable, may be generated. In a case where the comparison value is determined not to be equal to or greater than the threshold, a second indicator, which indicates that the determined the at least one of the first indication and the second indication is unreliable, may be generated. The comparison value may be a maximum value of a calculated cross-correlation between the first OCT image and the second OCT image. Other similarity measures that could alternatively be used are sum of squared differences, mutual information, normalised mutual information, and Kullback Leibler distance, for example.

In some other example embodiments, the computer-implemented method may further comprise calculating, for each set of a plurality of different sets of two or more OCT images of the sequence of OCT images, a respective comparison value being either (i) a respective value of an image quality metric calculated based on an amplitude component of at least one of the OCT images in the set, or (ii) a respective value of an image similarity metric that provides a measure of a degree of similarity between images, the value of the image quality metric being calculated based on amplitude components of at least two of the OCT images in the set. The method further comprises comparing each comparison value with a threshold to determine whether the comparison value is equal to or greater than the threshold. For each set of the plurality of different sets of OCT images, for which set the calculated comparison value is determined to be equal to or greater than the threshold, phase components of the OCT images in the set are processed to generate a respective velocity profile that is indicative of the distribution. Furthermore, for each set of the plurality of different sets of OCT images, for which set the calculated comparison value is determined not to be greater than the threshold, a respective velocity profile, which indicates zero velocity at all positions along the axial direction in the velocity profile, is generated. A concatenation of the calculated velocity profiles is then generated, such that the concatenation of the velocity profiles is indicative of how the distribution changes over time, and respective portions of the concatenation of the velocity profiles, which portions have the same position (e.g. one of the first position and the second position indicated by the generated first or second indication) along the axial direction, are integrated to generate data indicating an optical path length variation over time at the position along the axial direction. The generated data may comprise one or more sets of equal consecutive values, and the method may further comprise smoothing the generated data by replacing one or more values in at least one set of the one or more sets of equal consecutive values with one or more estimated values calculated based on neighbouring values that neighbour the set of equal consecutive values in the generated data.

In yet other example embodiments, the computer-implemented method may further comprise calculating, for each set of a plurality of different sets of two or more OCT images of the sequence of OCT images, a respective comparison value being either (i) a respective value of an image quality metric calculated based on an amplitude component of at least one of the OCT images in the set or (ii) a respective value of an image similarity metric that provides a measure of a degree of similarity between images, the value of the image quality metric being calculated based on amplitude components of at least two of the OCT images in the set. In these example embodiments, the method further comprises comparing each comparison value with a threshold to determine whether the comparison value is equal to or greater than the threshold. Some of the comparison values may be determined to be greater than or equal to the threshold, while other comparison values may be determined to be smaller than the threshold. For each set of the plurality of different sets of OCT images, for which set the calculated comparison value is determined to be equal to or greater than the threshold, phase components of the OCT images in the set are processed to generate a respective velocity profile that is indicative of the distribution. For each set of the plurality of different sets of OCT images, for which set the calculated comparison value is determined not to be greater than or equal to the threshold, a respective velocity profile that is indicative of the distribution is generated (estimated) based on a respective velocity profile calculated for each set of one or more other sets of the plurality of different sets of OCT images. A concatenation of the generated velocity profiles is then generated, such that the concatenation of the velocity profiles is indicative of how the distribution changes over time. Respective portions of the concatenation of the velocity profiles, which portions have the same position along the axial direction, are integrated to generate data indicating an optical path length variation over time at the position along the axial direction.

In further example embodiments, the computer-implemented method may further comprise calculating, for each set of a plurality of different sets of two or more OCT images of the sequence of OCT images, a respective comparison value being either (i) a respective value of an image quality metric calculated based on an amplitude component of at least one of the OCT images in the set or (ii) a respective value of an image similarity metric that provides a measure of a degree of similarity between images, the value of the image quality metric being calculated based on amplitude components of at least two of the OCT images in the set. In these example embodiments, each comparison value is compared with a threshold to determine whether the comparison value is equal to or greater than the threshold. Some of the comparison values may be determined to be greater than or equal to the threshold, while other comparison values may be determined to be smaller than the threshold. For each set of the plurality of different sets of OCT images, for which set the calculated comparison value is determined to be equal to or greater than the threshold, phase components of the OCT images in the set are processed to generate a respective velocity profile that is indicative of the distribution. For each set of the plurality of different sets of OCT images, for which set the calculated comparison value is determined not to be greater than or equal to the threshold, a respective velocity profile that is indicative of the distribution is generated based a respective velocity profile calculated for each set of one or more other sets of the plurality of different sets of OCT images. A concatenation of the generated velocity profiles is then generated such that the concatenation of the velocity profiles is indicative of how the distribution changes over time, and respective portions of the concatenation of the velocity profiles, which portions have the same position along the axial direction, are integrated to generate data indicating an optical path length variation over time at the position along the axial direction.

The respective comparison value calculated for each set may be a respective maximum value of a calculated cross-correlation between the at least two of the OCT images in the set. Other similarity measures that could alternatively be used are sum of squared differences, mutual information, and normalised mutual information, for example.

In example embodiments wherein the concatenation is generated, a respective indication of an optical path length variation over time at each of at least one of the first position along the axial direction and the second position along the axial direction indicated by the at least one of the first indication and the second indication may be determined by integrating respective portions of the concatenation at the at least one of the first position along the axial direction and the second position along the axial direction.

In any of the foregoing, the mentioned layer of the retina may comprise an outer segment of photoreceptor cells.

Furthermore, in any of the foregoing, the computer-implemented method may further comprise processing the phase component of the first OCT image and the phase component of the second OCT image, before calculation of the velocity profile, to compensate for a bulk motion of the common portion of the retina during acquisition of the sequence of OCT images by a Fourier-domain OCT imaging system after stimulation of the common portion by the optical stimulus.

There is also provided, in accordance with a second example aspect herein, a computer program comprising computer-readable instructions which, when executed by a processor, cause the processor to perform the method of the first example aspect or any of its example implementations and embodiments set out above. The computer program may be stored on a non-transitory computer-readable storage medium (such as a computer hard disk or a CD, for example) or carried by a computer-readable signal.

There is also provided, in accordance with a third example aspect herein, a data processing apparatus arranged to process a phase component of a first OCT image and a phase component of a second OCT image of a sequence of OCT images of a common portion of a retina of an eye acquired by a Fourier-domain optical coherence tomography, OCT, imaging system after stimulation of the common portion by an optical stimulus, wherein the common portion comprises a layer of the retina whose thickness changed in response to the optical stimulus during acquisition of the sequence of OCT images, to determine at least one of a first indication of a first position along an axial direction in the OCT images of a first boundary of the layer and a second indication of a second position along the axial direction in the OCT images of a second boundary of the layer. The data processing apparatus is arranged to perform the method of the first example aspect or any of its example implementations and embodiments set out above. The data processing apparatus may comprise a processor and a storage device storing computer-readable instructions which, when executed by the processor, cause the processor to perform the method of the first example aspect or any of its example implementations and embodiments set out above. Alternatively, the data processing apparatus may be implemented in non-programmable hardware, such as an ASIC, an FPGA or other integrated circuit configured to perform any of these methods.

There is also provided, in accordance with a fourth example aspect herein, a Fourier-domain OCT imaging system comprising the data processing apparatus of the third example aspect set out above.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of a system comprising a Fourier-domain OCT imaging system 30 and a data processing apparatus 100 according to example embodiments herein.
Figure 2 is a schematic illustration of an example implementation of the data processing apparatus 100 of the example embodiment in programmable signal processing hardware.
Figure 3 is a flow diagram illustrating a method according to an example embodiment, by which phase components of OCT images from a sequence of OCT images of a common portion of a retina acquired after optical stimulation of the common portion are processed to identify a boundary of the layer.
Figure 4A illustrates a sinusoidal bulk oscillation added to oscillatory movements of two model retinal layers.
Figure 4B shows the effect of removing the bulk motion on the variation with time of the phase angle of light reflected from the two model retinal layers.
Figure 5 illustrates ORG contributors of the two model retinal layers that are set apart from each other and do not overlap.
Figure 6 illustrates the magnitude of the model retina ORG response for the case where no compensation for bulk motion is provided.
Figure 7A illustrates the phase of the model retina ORG response for the case where no compensation for bulk motion is provided.
Figure 7B illustrates the phase of the ORG response for the case where bulk motion has been compensated for.
Figure 8 illustrates PCA components obtained by applying a PCA algorithm in MATLAB^{™} to the ORG response calculated for the case where bulk motion has been compensated for.
Figure 9 illustrates absolute values of the principal component coefficients returned by the PCA algorithm.
Figure 10 illustrates ORG contributors of two retinal layers in a variant, wherein the ORG contributors of the two retinal layers partially overlap each other.
Figure 11 illustrates PCA components obtained by applying the PCA algorithm to the ORG response calculated using the ORG contributors of Figure 10, for the case where bulk motion has been compensated for
Figure 12 illustrates absolute values of the principal component coefficients returned by the PCA algorithm in the variant.
Figure 13 is a flow diagram illustrating a method by which the data processing apparatus 100 of the example embodiment may determine whether the determined first indication Z_{B-1} and/or the second indication Z_{B-2} (as the case may be) is reliable or unreliable, and generate an indicator indicative of the determined reliability/unreliability.
Figure 14A shows a first pair of B-scans that are highly correlated.
Figure 14B shows a calculated cross-correlation for the B-scans in Figure 14A.
Figure 15A shows a second pair of B-scans that are not highly correlated.
Figure 15B shows a calculated cross-correlation for the B-scans in Figure 15A.
Figure 16 is a flow diagram illustrating a process by which the data processing apparatus 100 of a second example embodiment herein may processes pairs of adjacent B-scans in a sequence of B-scans to generate ORG data that is indicative of the response of the retina to an applied light stimulus.
Figure 17 shows an example of a concatenation of the velocity profiles that may be generated by the data processing apparatus in accordance with process S180 of Figure 16.
Figure 18 shows a plot of changes in optical path length ΔOPL over time, which have been calculated by the data processing apparatus of the second example embodiment for a selected position in the velocity profiles of a concatenation of velocity profiles.
Figure 19 shows a plot of an example ORG signal generated by the data processing apparatus 100 of an example embodiment herein.
Figure 20 shows a result of smoothing the ORG signal in Figure 19.
Figure 21 is a flow diagram illustrating a process by which a data processing apparatus in accordance with the second example embodiment herein may processes adjacent OCT images in a sequence of OCT images to generate ORG data that is indicative of the response of the retina to an applied light stimulus.
Figure 22 is a flow diagram illustrating a process by which the data processing apparatus of a third example embodiment may processes adjacent OCT images in a sequence of OCT images to generate ORG data that is indicative of the response of the retina to an applied light stimulus.

### [Detailed Description of Example Embodiments]

To analyse an ORG response, different layers of the retina are often selected and the change in optical path length (OPL) is calculated between the layers of interest. Where OCT images in the form of B-scans are processed, a flattening algorithm is typically used to distort the B-scans so that the retinal layers lie at a constant depth in the distorted B-scans. The retinal layers are then usually either found by manual selection or by automatically finding the depth location of the intensity peaks in the B-scans that correspond to the layers. The manual approach is undesirable as it requires input from an operator, and can introduce a bias and slow down the analysis, and also requires operator knowledge. Although the automatic finding of intensity peaks usually has a high success rate, it can be problematic when the OCT signal is weak and the different layers are not well-defined, particularly in scans with low axial resolution, scans in the periphery of the retina, or where the layers of interest have weak intensities (e.g. the inner ganglion layers).

The OCT data processing methods described herein may solve some or all these problems, and do not rely on identifying OCT signal intensity peaks, which can be difficult in cases where the OCT data has poor axial resolution or is noisy. At least some of the OCT data processing methods described herein may allow layer segmentation to be carried out effectively on OCT data from FD-OCT systems with poor axial resolution which may not be sufficient to show the COST, rod outer segment (ROST) and/or retinal pigment epithelium (RPE) intensity peaks, for example, and allow such boundaries to be identified. Furthermore, they may be advantageous in allowing ORG responses to be extracted from different regions of the retina, such as the ganglion cells, which typically provide weak OCT signal intensities. At least some of the OCT data processing methods described herein also allow for a conventional optical path length response between photoreceptor layers to be found using a conventional point-scan FD-OCT system with sequential registration and without adaptive optics or tracking systems.

### [First Example Embodiment]

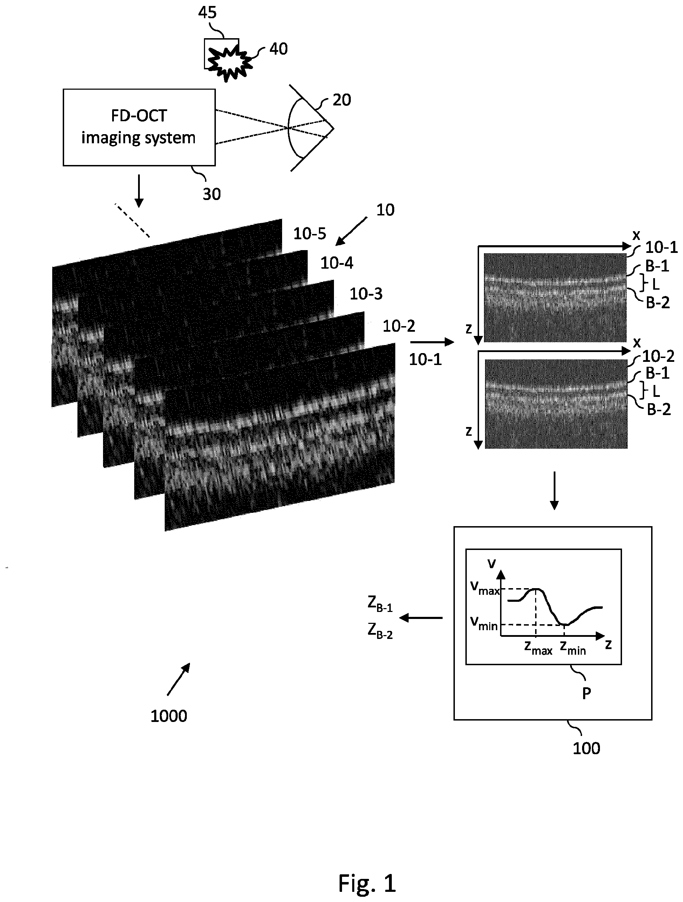

Figure 1 is a schematic illustration of a data processing apparatus 100 according to a first example embodiment. The data processing apparatus 100 is arranged to process complex OCT data generated by a Fourier-domain OCT (FD-OCT) imaging system. More specifically, the data processing apparatus 100 is arranged to process a phase component (phase information) of the first OCT image and a phase component of a second OCT image of a sequence of OCT images of a common portion of a retina of an eye 20 acquired by a phase-resolved FD-OCT imaging system 30 after stimulation of the common portion of the retina by an optical stimulus 40, which is generated by an optical stimulus source 45 such as a light-emitting diode (LED). The sequence of OCT images 10 was acquired by the FD-OCT imaging system 30 while the thickness of a layer L in the common portion of the retina changed in response to the light stimulus 40, and may form part of a wider sequence of OCT images acquired by the FD-OCT imaging system 30 that also includes OCT images acquired before application of the optical stimulus 40.

The FD-OCT imaging system 30 may, as in the present example embodiment, be a swept-source OCT (SS-OCT) system. However, the FD-OCT imaging system 30 need not be provided in this form and may, for example, take the alternative form of a spectral-domain OCT (SD-OCT). More generally, an example embodiment may be provided as any form of phase-resolved FD-OCT imaging system that can generate complex OCT data, i.e. Fourier transforms of respective spectral interferograms (interference spectra) representing complex A-scan information obtained for each scan location at which an OCT measurement is made during the scan. Such complex OCT data encodes phase information from acquired OCT measurements that can be used by the data processing apparatus 100 as described herein to identify one or both boundaries of a layer of the retina of the eye 20.

The FD-OCT imaging system 30 may include well-known components, including a scanning system, a light detector, OCT data processing hardware, and a light beam generator (not shown). The scanning system may be arranged to perform a one- and/or two-dimensional point-scan of a light beam across the retina, and collect light which has been scattered by the retina during the point scan. The scanning system is therefore arranged to acquire A-scans at respective scan locations that are distributed across a surface of the retina, by sequentially illuminating the scan locations with the light beam, one scan location at a time, and collecting at least some of the light scattered by the retina at each scan location. The scanning system may acquire OCT images in the form of repeat B-scans by performing the point-scan using a linear scan pattern, wherein a set of overlapping scan lines are followed during the scan. Although the scanning system is arranged to acquire repeat B-scans by performing point-scans in the present example embodiment, the scanning system may alternatively be arranged to acquire the repeat B-scans by performing line-scans in other example embodiments, using hardware well-known to those versed in the art. The FD-OCT imaging system 30 may alternatively be arranged to acquire OCT images in the form of C-scans by performing point-scans or a line-scans using techniques well-known to those versed in the art, or by employing a full-field set-up.

The first OCT image mentioned above may, as in the present example embodiment, be in the form of a first OCT B-scan 10-1, and the second OCT image may be in the form of a second OCT B-scan 10-2, as shown in Figure 1. However, the form of the first and second OCT images is not so limited, and the first and second OCT images may be respective OCT C-scans, for example. The first B-scan 10-1 and the second B-scan 10-2 of the common portion of the retina may, as in the present example embodiment, be next to each other in a sequence of OCT B-scans 10 or they may be separated by n intervening B-scans, where n ≥ 1 but small enough for the B-scans to have a sufficiently high degree of phase correlation to one another, owing to the absence of significant relative movement of the eye 20 with respect to the FD-OCT imaging system 30. Furthermore, as explained in more detail below, the first B-scan 10-1 may be processed as one of a first set of adjacent B-scans in the sequence of B-scans 10, and the second B-scan 10-2 may be processed as one of a second set of adjacent B-scans in the sequence of B-scans 10, where the first and second sets of adjacent OCT images may or may not have one or more OCT images in common.

The layer L may, as in the present example embodiment, be the photoreceptor outer segment (OS) of the retina, which contains rod and cone cells and functions to transduce an absorbed visible light signal into a change of membrane potential. The photoreceptor OS is particularly well suited for ORG measurements; the photoreceptors therein are long and narrow, behave like optical waveguides and provide relatively strong reflections from each end (i.e. the IS/OS junction and the COST). The phase changes between these reflections are used to quantify the optical path length change of the OS. However, the layer L need not comprise (or be limited to) the photoreceptor OS, and may alternatively be another one or more layers of the retina that produce a measurable change in thickness when stimulated, such as the ganglion cell layer/inner plexiform layer (GCL/IPL). The GCL and IPL would provide much weaker reflections that are nevertheless detectable, particularly where steps are taken to suppress motion artefacts (see, for example "Simultaneous functional imaging of neuronal and photoreceptor layers in living human retina" by C. Pfäffle et al., Optic Letters, Vol. 44, No. 23, pages 5671-5674 (1 December 2019)).

As described in more detail below, the processing of the phase component of the first B-scan 10-1 and the phase component of the second B-scan 10-2 by the apparatus 100 results in the determination by the apparatus 100 of one or both of a first indication Z_{B-1} of a first position along an axial direction (z-axis direction) z in the B-scans 10 of a first boundary (edge) B-1 of a layer L of the retina, and a second indication Z_{B-2} of a second position along the axial direction z in the B-scans 10 of a second boundary B-2 of the layer L. The axial direction (or z-axis direction) in the B-scans is the direction in any of the B-scans along which the elements of a component A-scan of the B-scan are arrayed). Where the layer L is the photoreceptor OS, as in the present example embodiment, the first boundary B-1 of the layer L is the IS/OS junction, and the second boundary B-2 of the layer L is the COST. Each of the first indication Z_{B-1} and the second indication Z_{B-2} may, as in the present example embodiment, provide a respective row index for the row of the B-scans 10 at whose position the respective boundary is located, although the indications Z_{B-1} and Z_{B-2} may alternatively be scaled versions of these indices, for example (e.g. in cases where the depth of the IS/OS function and the COST from the retinal surface is required).

As described in more detail below, the data processing apparatus 100 is arranged to process at least part of the phase component of the first B-scan 10-1 and at least part of the phase component of the second B-scan 10-2 to calculate a tissue velocity profile P comprising values of tissue velocity whose variation with position in the velocity profile is indicative of a distribution of velocity among points along the axial direction z in common portion of the retina (as illustrated in Figure 1, for example). The velocity profile P may, as in the present example embodiment, thus be a two-dimensional data structure in which values of the tissue velocity v are associated with corresponding values of a position. The velocity profile P may more generally be a two-dimensional data structure in which values (of a calculated change in phase or optical path length, for example) that are indicative of the velocity v are associated with corresponding values of a position. The data processing apparatus 100 is arranged to determine one or both of the first indication Z_{B-1} and the second indication Z_{B-2} based on the calculated velocity profile P.

The data processing apparatus 100 may be provided in any suitable form, for example as a programmable signal processing hardware 200 of the kind illustrated schematically in Figure 2. The programmable signal processing hardware 200 comprises a communication interface (I/F) 210, for receiving the B-scans 10 (or OCT images of another form, such as C-scans) from the FD-OCT imaging system 30, and outputting one or both of the first indication Z_{B-1} and the second indication Z_{B-2} (as the case may be) and/or a graphical representation thereof on an OCT image (e.g. B-scan) being displaying on a display, such a computer screen or the like. The communication interface 210 may also output reliability indicators, which are described in more detail below. The signal processing hardware 200 further comprises a processor (e.g. a Central Processing Unit, CPU, and/or a Graphics Processing Unit, GPU) 220, a working memory 230 (e.g. a random-access memory) and an instruction store 240 storing a computer program 245 comprising the computer-readable instructions which, when executed by the processor 220, cause the processor 220 to perform the various functions of the data processing apparatus 100 described herein. The working memory 230 stores information used by the processor 220 during execution of the computer program 245. The instruction store 240 may comprise a ROM (e.g. in the form of an electrically erasable programmable read-only memory (EEPROM) or flash memory) which is pre-loaded with the computer-readable instructions. Alternatively, the instruction store 240 may comprise a RAM or similar type of memory, and the computer-readable instructions of the computer program 245 can be input thereto from a computer program product, such as a non-transitory, computer-readable storage medium 250 in the form of a CD-ROM, DVDROM, etc. or a computer-readable signal 260 carrying the computer-readable instructions. In any case, the computer program 245, when executed by the processor 220, causes the processor 220 to perform the functions of the data processing apparatus 100 described herein. Thus, the data processing apparatus 100 of the example embodiment may comprise a computer processor 220 and a memory 240 storing computer-readable instructions which, when executed by the processor 220, cause the processor 220 to process the phase component of the first B-scan 10-1 and the phase component of the second B-scan 10-2 to calculate the velocity profile P, and to determine the first indication Z_{B-1} and/or the second indication Z_{B-2} based on the calculated velocity profile P.

It should be noted, however, that the data processing apparatus 100 may alternatively be implemented in non-programmable hardware, such as an ASIC, an FPGA or other integrated circuit dedicated to performing the functions of the data processing apparatus 100 described herein, or a combination of such non-programmable hardware and programmable signal processing hardware 200 as described above with reference to Figure 2.

The data processing apparatus 100 may be provided as a stand-alone product or as part of a system 1000 comprising the optical stimulus source 45, which is arranged to provide the optical stimulus 40, and the FD-OCT imaging system 30, which is arranged to acquire the sequence of OCT images 10 of the common portion of the retina of the eye 20 after stimulation of the common portion by the optical stimulus 40. The data processing apparatus 100 is arranged to process a phase component of a first OCT image 10-1 and a phase component of a second OCT image 10-2 of the sequence of OCT images 10 acquired by the FD-OCT imaging system 30 using the techniques described herein.

Figure 3 is a flow diagram illustrating a method according to an example embodiment, by which phase components of B-scans 10-1 and 10-2 from the sequence of B-scans 10 of the common portion of a retina acquired after optical stimulation of the common portion are processed by the data processing apparatus 100 to identify one or both of the first boundary B-1 and the second boundary B-2 of the layer L which may, as in the present example embodiment, be the IS/OS junction and the COST, respectively.

In process S10 of Figure 3, the data processing apparatus 100 processes the phase component of the first B-scan 10-1 and the phase component of the second B-scan 10-2 to calculate a velocity profile P indicative of a distribution, along the axial direction z, of velocity v within the common portion of the retina. The data processing apparatus 100 thus calculates a velocity profile P comprising values of velocity v (or values of a change in phase or optical path length, ΔOPL, for example) whose variation with position in the velocity profile is indicative of a distribution of velocity v of portions of the retina (within the common portion) at respective points lying on a line aligned with the axial direction. The velocity profile P is thus a two-dimensional data structure in which values of the velocity v (or values of a variable indicative thereof, such as ΔOPL or a change in phase, Δϕ) are associated with corresponding values of the position, and this data structure may be visualised in the form of a plot of velocity v (or Δϕ or ΔOPL) as a function of position z, as shown schematically in Figure 1.

Before calculating the velocity profile P, the data processing apparatus 100 may, as in the present example embodiment, process the phase component of the first OCT image 10-1 and the phase component of the second OCT image 10-2 to compensate for a bulk motion of the common portion of the retina during acquisition of the sequence of OCT images 10 by the Fourier-domain OCT imaging system 30 after stimulation of the common portion by the optical stimulus 40. Compensating for bulk motion was found to increase the reliability of the velocity-based layer segmentation techniques described herein.

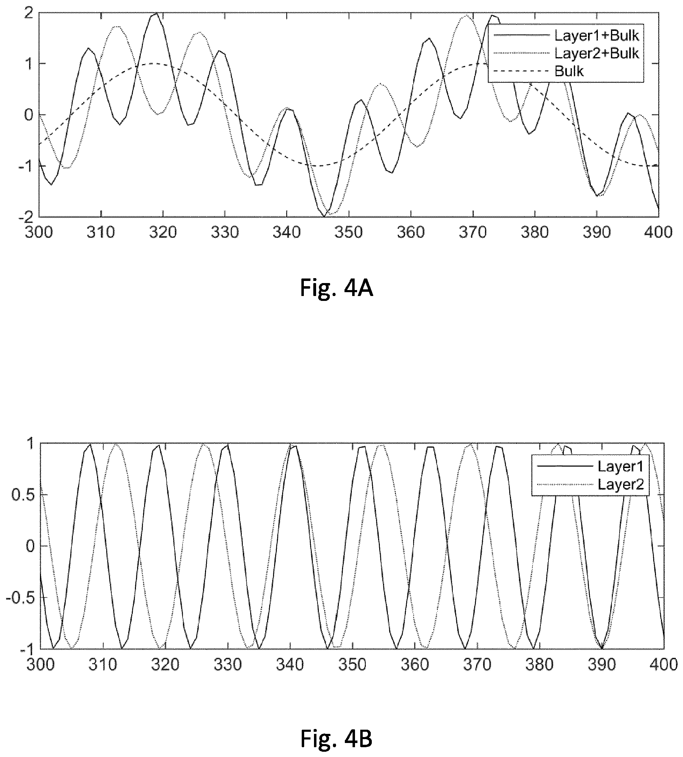

Figure 4A illustrates a sinusoidal bulk oscillation added to oscillatory movements of two model retinal layers. In this example, the bulk movement is modelled by adding a phase exp(icp), where *φ* represents a fraction of a wavelength (half wavelength movement is 2π in double pass reflection). Figure 4A shows how the phase angle of light reflected from the first model retinal layer (Layer 1) and the second model retinal layer (Layer 2) varies over time whilst the layers are subject to the common bulk motion as well as their individual oscillations (i.e. the plots labelled "Layer 1 + Bulk" and "Layer 2 + Bulk" in Figure 4A). Figure 4A also shows how the phase angle of light reflected from either model retinal layer varies over time whilst the layer is subject to the bulk motion only (i.e. the plot labelled "Bulk" in Figure 4A). Figure 4B shows the effect of removing the bulk motion on the variation of the phase angle of light reflected from the two model retinal layers with time.

The MATLAB^{™} code used to generate the plots in Figures 4A and 4B is as follows:

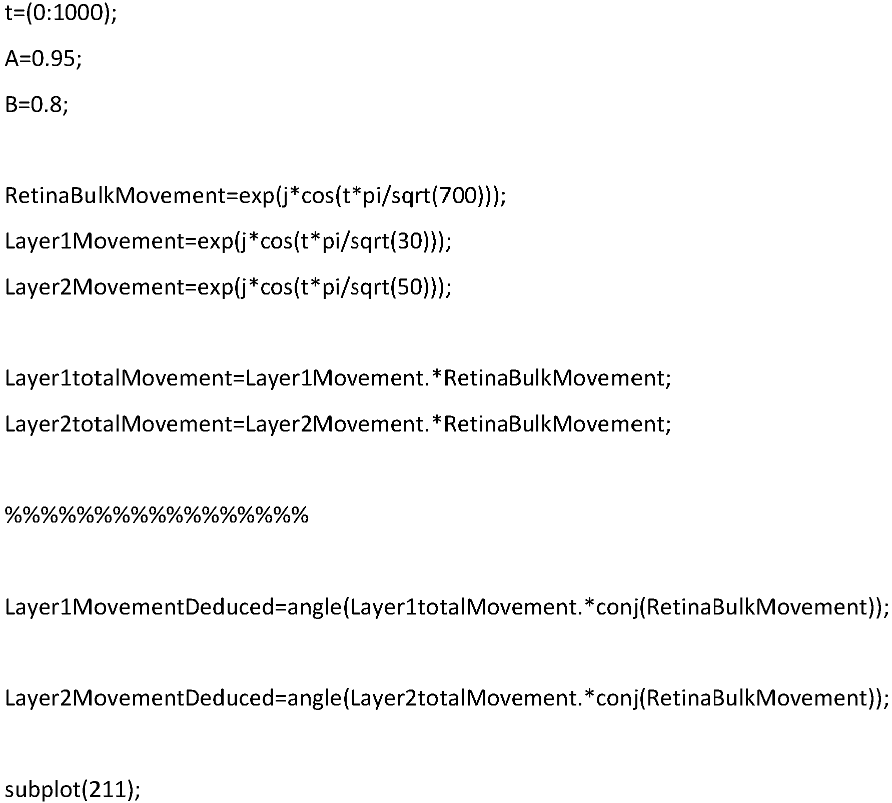

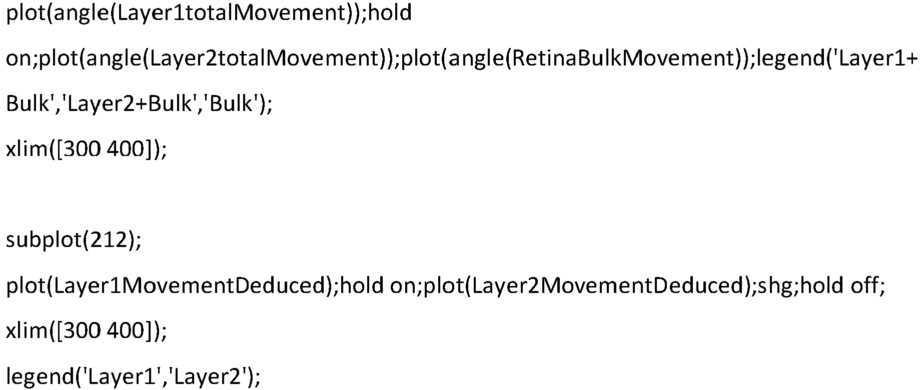

Referring again to Figure 3, in process S20, the data processing apparatus 100 determines one or both of the first indication Z_{B-1} and the second indication Z_{B-2} based on the calculated velocity profile P. Thus, in contrast to conventional OCT signal amplitude-based layer segmentation (whether performed manually by the user or automatically) commonly employed in ORG, a velocity-based layer segmentation is used in the present example embodiment.

An example of a process by which the data processing apparatus 100 may perform S10 of Figure 3 will now be described, which is based on the velocity-based ORG technique described in Kari V. Vienola, et al., "Velocity-based optoretinography for clinical applications," Optica 9, 1100-1108 (2022).

In process S10 of Figure 3, the data processing apparatus 100 may, as in the present example embodiment, first flatten the first B-scan 10-1 and the second B-scan 10-2 such that the IS/OS and COST reflections lie at substantially the same height for each A-scan in each of the B-scans. The data processing apparatus 100 may then register the second B-scan 10-2 with respect to the first B-scan 10-1. The phase data of the two B-scans for each spatial coordinate pair (i.e. the phase data at the same (x, z) coordinates in the B-scans) may then be unwrapped in the temporal dimension to minimise the magnitude of the difference in phase between the data sets of the two B-scans 10-1 and 10-2. After unwrapping and processing the phase components of the first OCT image 10-1 and the second OCT image 10-2 to compensate for the bulk motion of the retina during imaging, a difference between corresponding phase values in the B-scans 10-1 and 10-2 is calculated for each spatial location specified by a corresponding coordinate pair, and the instantaneous velocity for the spatial location is then calculated using the difference between the acquisition times of the B-scans. These instantaneous velocities may, as in the present example embodiment, be averaged in the lateral dimension (i.e. along the x-axis) to give instantaneous, depth-dependent measures of velocity along the z-axis, i.e. a one-dimensional velocity profile P of the kind illustrated schematically in Figure 1, which is indicative of a distribution of velocity v along the axial direction (z-axis) within the common portion of the retina that is covered by the first and second B-scans 10-1 and 10-2. The B-scan amplitudes (if desired) can also be averaged in the lateral dimension (x-axis) to give instantaneous, depth-dependent measures of backscattering. It should be noted that averaging in the lateral dimension is not required, and a two-dimensional velocity profile may alternatively be generated, which is indicative of the distribution of velocity v along both the axial direction (z-axis) and the lateral direction (x-axis) within the common portion of the retina that is covered by the first B-scan 10-1 and the second B-scan 10-2.

Once the boundaries of the OS have been identified as described below, their respective velocities can be extracted and the difference between them provides the rate of the contraction/elongation of the OS at the time the B-scans 10-1 and 10-2 were acquired by the FD-OCT imaging system 30, which can be used to generate ORG data indicative of the response of the retina in the common portion to the applied stimulus.

In process S20 of Figure 3, the data processing apparatus 100 may determine the first indication Z_{B-1} of the first position along the axial direction z in the B-scans 10 of the first boundary B-1 of the layer L by determining, as the first indication Z_{B-1}, a first position zₘₐₓ in the velocity profile P corresponding to a maximum of the velocity, vₘₐₓ, in the velocity profile P. The maximum here may the global maximum of velocity in the velocity profile P or a maximum within a predefined portion of the velocity profile P, within which portion the OS is expected to be located. The predefined portion can be defined relative to the top surface of the retina from known physiology of the eye, for example. Similarly, the data processing apparatus 100 may determine the second indication Z_{B-2} of the second position along the axial direction z in the B-scans 10 of the second boundary B-2 of the layer L in process S20 of Figure 3 by determining, as the second indication Z_{B-2}, a second position zₘᵢₙ in the velocity profile P corresponding to a minimum of the velocity, vₘᵢₙ, in the velocity profile P. The minimum here may the global minimum of velocity in the velocity profile P or a minimum within the predefined portion in which the OS is expected to be located, as described above.

Thus, the velocity v in a portion of the velocity profile P corresponding to the photoreceptor OS in the retina varies (usually monotonically) from a maximum velocity vₘₐₓ at a first position zₘₐₓ in the velocity profile P corresponding to the IS-OS junction at a given location on the retina to a minimum velocity vₘᵢₙ at a second position zₘᵢₙ in the velocity profile P corresponding to the COST at that location on the retina, and the first indication Z_{B-1} indicates the first position zₘₐₓ in the velocity profile P of the maximum velocity vₘₐₓ, and the second indication Z_{B-2} indicates the second position zₘᵢₙ in the velocity profile P of the minimum velocity vₘᵢₙ.

The position(s) of one or both of the boundaries of the OS or other layer L of the retina, which position(s) change in response to applied optical stimuli (for example, the position of the rod outer segment tips (ROST) or the retina pigment epithelium (RPE)) may be determined using the velocity-based approach to segmentation described herein. For example, the velocity profile P may have a further maximum followed by a further minimum going along the z-axis in Figure 1 for z > zₘᵢₙ, which corresponds to a change in length of rods in response to the applied stimulus. In the present example embodiment, the data processing apparatus 100 determines both the first position zₘₐₓ and the second position zₘᵢₙ in the velocity profile P by firstly calculating, for each combination of a candidate first position, zᵢ, in the velocity profile P and a candidate second position, zⱼ, in the velocity profile P of all combinations (zᵢ, zⱼ) of candidate first and second positions, a respective value of a difference, Dᵢⱼ, between a respective velocity vᵢ at the candidate first position zᵢ and a respective velocity vⱼ at the candidate second position zⱼ in the combination (zᵢ, zⱼ). The data processing apparatus 100 then identifies, as the first position zₘₐₓ and the second position zₘᵢₙ, a candidate first position zᵢ and a candidate second position zⱼ of a combination in the plurality of combinations for which the calculated value of the difference Dᵢⱼ is greatest among the calculated values of the difference.

In a variant of the present example embodiment, the data processing apparatus 100 determines both the first position zₘₐₓ and the second position zₘᵢₙ in the velocity profile P by firstly calculating, for each combination of a first candidate first position, zᵢ, in the velocity profile P and a candidate second position, zⱼ, in the velocity profile P of all combinations (zᵢ, zⱼ) of candidate first and second positions, a respective value of a difference between an average of respective velocities vᵢ₋₂, vᵢ₋₁, vᵢ, vᵢ₊₁ and vᵢ₊₂ at a set of five adjacent positions zᵢ₋₂, zᵢ₋₁, zᵢ, zᵢ₊₁ and zᵢ₊₂ that are centred on (but may otherwise include) the candidate first position zᵢ in the combination, and an average of respective velocities vⱼ₋₂, vⱼ₋₁, vⱼ, vⱼ₊₁ and vⱼ₊₂ at a set of five adjacent positions zⱼ₋₂, zⱼ₋₁, zⱼ, zⱼ₊₁ and zⱼ₊₂ that are centred on (but may otherwise include) the candidate second position zⱼ in the combination. Although the number of adjacent positions is five in the present example embodiment, it will be appreciated that this has been given by way of an example only, and there may be a different number of adjacent positions (in general, two or more) in other embodiments. The data processing apparatus 100 then identifies, as the first position zₘₐₓ and the second position zₘᵢₙ, a candidate first position zᵢ and a candidate second position zⱼ of a combination in the plurality of combinations for which the calculated value of the difference Dᵢⱼ is greatest among the calculated values of the difference. Finding the maximum of the difference Dᵢⱼ gives the maximum change in optical path length and thus indicates where the neurons experience the largest change due to the stimulus.

In a further variant of the present example embodiment, the data processing apparatus 100 is arranged to determine the first indication Z_{B-1} and/or the second indication Z_{B-2} by processing the calculated velocity profile P using a cluster analysis algorithm or a dimensionality reduction algorithm. These approaches to processing the velocity profile P can be used to identify variations therein relating to retinal layers that may be hidden by other layers and/or anatomical features. The dimensionality reduction algorithm may be of one of several different kinds known to those versed in the art, and may be based on a machine learning (ML) approach. The dimensionality reduction algorithm may comprise a principal component analysis (PCA) algorithm, an independent component analysis (ICA) algorithm, a linear discriminant analysis (LDA) algorithm or a non-negative matrix factorisation (NMF) algorithm, for example.

Where the dimensionality reduction algorithm is a PCA algorithm, as in the present variant, one or both of the first indication Z_{B-1} or the second indication Z_{B-2} may be determined using principal components that have been determined by applying the PCA algorithm to the calculated velocity profile P. For a better understanding of how this can be done, some illustrative examples of how PCA may be used in MATLAB^{™} to identify data elements of two model retinal layers that contribute to an ORG response (these data elements hereinafter being referred to as "ORG contributors"), and to localize these ORG contributors, as well as identify which of the layers they belong to, thus allowing retinal layers that exhibits an ORG response to be demarcated, will now be described with reference to Figures 5 to 12.

In these examples, a single A-scan is considered for simplicity, and the ORG contributors are provided at multiple locations in the A-scan for each of the two layers. In a first of these examples, the ORG contributors of the two retinal layers are set apart from each other and do not overlap, as illustrated in Figure 5, where the x-axis represents the location within the A-scan of a contributor, and the y-axis represents the strength of the contributor. The MATLAB^{™} code used to generate the plot in Figure 5 is as follows: $I 1 = transpose \left(\left[0 0.15 0.4 0.25 0 0 0 0 0 0\right]\right) ;$ $I 2 = transpose \left(\left[0 0 0 0 0 0 0.15 0.5 0.3 0\right]\right) ;$
figure;
plot(l1);hold on; plot(l2);hold off;
legend('Retina element 1', 'Retina element 2')

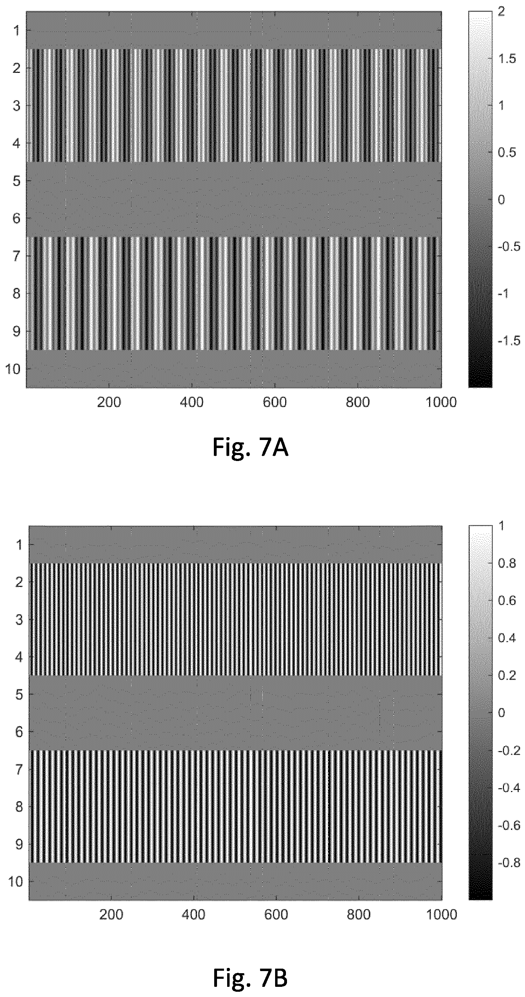

The phases of model retinal layer 1 and model retinal layer 2 are then assigned to the ORG contributors shown in Figure 5. The magnitude of the (complex) model retina ORG response is shown in Figure 6 for the case where no compensation for bulk motion is provided, while the phase of that ORG response is shown in Figure 7A. The MATLAB^{™} code used to generate Figures 6 and 7A is as follows: $RetinaORG = I 1 * Layer 1 totalMovement + I 2 * Layer 2 totalMovement ;$
figure;
imagesc(abs(RetinaORG));colormap gray;title('OCT amplitude')
imagesc(angle(RetinaORG)); colormap parula;colorbar;

Figure 7B illustrates the phase of the ORG response for the case where bulk motion has been compensated for. The MATLAB^{™} code usd to generate Figure 7B is as follows: $RetinaORGComp = RetinaORG . * conj \left(RetinaBulkMovement\right) ;$ imagesc(angle(RetinaORGComp)); colormap parula;colorbar;

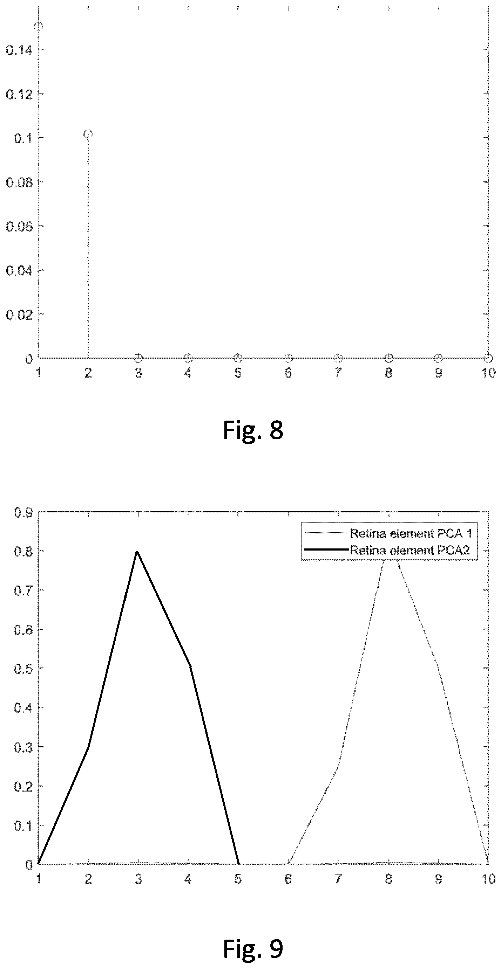

PCA is then used to automatically detect the main ORG contributors and identify their locations within the A-scan. Applying the PCA algorithm in MATLAB^{™} to the (complex) ORG response calculated for the case where bulk motion has been compensated for (the phase of the ORG response being illustrated in Figure 7B) yields two PCA components, as illustrated in Figure 8. The MATLAB^{™} code used to generate Figure 8 is as follows:
[COEFF2, SCORE2, LATENT2]=pca((RetinaORGComp_t));
stem(LATENT2);

The absolute values of the principal component coefficients (i.e. loadings) returned by the PCA algorithm are plotted in Figure 9. The MATLAB^{™} code used to generate Figure 9 is as follows:
plot(abs(COEFF2(:,1:2)));
legend('Retina element PCA 1', 'Retina element PCA2');

In Figure 9, the ORG contributors associated with Layer 1 (labelled "Retina element PCA 1") appear to the right of those associated with Layer 2 (labelled "Retina element PCA 2"), which contrasts with Figure 5, wherein the ORG contributors associated with Layer 1 (labelled "Retina element 1") appear to the left of those associated with Layer 2 (labelled "Retina element 2"). The ORG contributor values in Figure 9 also differ slightly from those in Figure 5. Nevertheless, the PCA algorithm has identified where the main contributors come from and has grouped them.

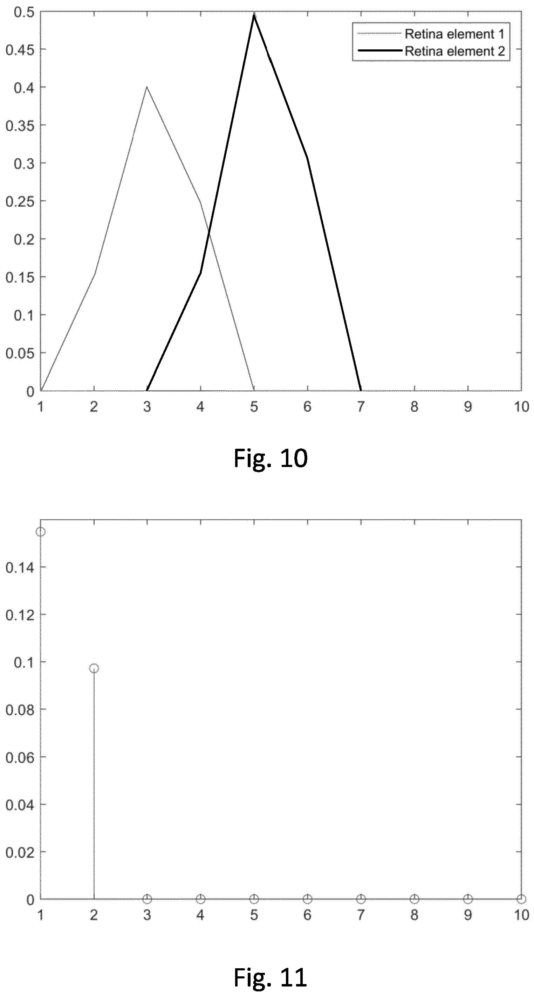

In the above example, there is no spatial overlap of the ORG contributors associated with Layer 1 and Layer 2, as can be seen in Figure 5. However, in a more realistic situation, a location in the A-scan could collect two ORG contributors, and the application of PCA to a variant of the above example, wherein the ORG contributors of the two retinal layers partially overlap each other, will now be described with reference to Figures 10 to 12.

Figure 10 shows the ORG contributors of the two retinal layers in the variant mentioned above, where the x-axis once again represents the location within the A-scan of a contributor, and the y-axis represents the strength of the contributor. The MATLAB^{™} code used to generate Figure 10 is as follows: $I 1 b = transpose \left(\left[0 0.15 0.4 0.25 0 0 0 0 0 0\right]\right) ;$ $I 2 b = transpose \left(\left[0 0 0 0.15 0.5 0.3 0 0 0 0\right]\right) ;$
figure();
plot(l1b); hold on; plot(l2b);hold off;
legend('Retina element 1', 'Retina element 2')

PCA is then used to automatically detect the main ORG contributors and identify their locations within the A-scan. Applying the PCA algorithm in MATLAB^{™} to the ORG response calculated for the case where bulk motion has been compensated for again yields two PCA components, as illustrated in Figure 11. The MATLAB^{™} code used to generate Figure 11 is as follows: $RetinaORGb = I 1 b * Layer 1 totalMovement + I 2 b * Layer 2 totalMovement ;$ $RetinaORGCompb = RetinaORGb . * conj \left(RetinaBulkMovement\right) ;$ $RetinaORGComp_t = transpose \left(RetinaORGCompb\right) ;$
[COEFFb, SCOREb, LATENTb]=pca((RetinaORGCompb_t));
stem(LATENTb);

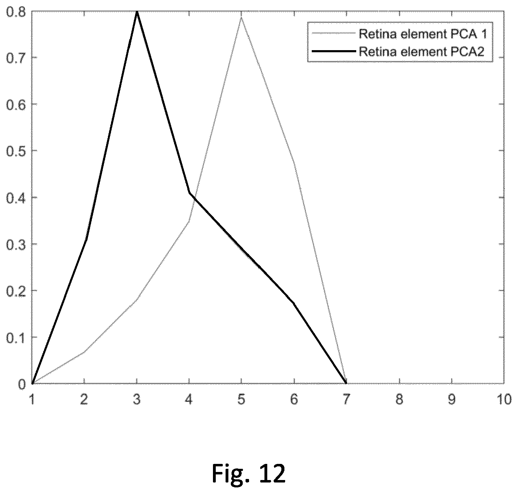

The absolute values of the principal component coefficients returned by the PCA algorithm are plotted in Figure 12. The MATLAB^{™} code used to generate Figure 12 is as follows:
plot(abs(COEFFb(:,1:2)));
legend('Retina element PCA 1', 'Retina element PCA2');

As can be seen in Figure 12, despite the layers being shown in inverted order and with slightly different values (as in the case of Figure 9), the PCA algorithm has once again identified where the main contributors come from and has grouped them.

In addition or as an alternative to determining the positions of both boundaries of the layer L using the velocity-based approach to segmentation described herein, the data processing apparatus 100 may provide the functionality of determining the position of one of the boundaries of the layer L using a conventional OCT signal amplitude-based approach and the remaining boundary using the velocity-based approach, as will now be described.

In a variant of the example embodiment having such functionality, the data processing apparatus 100 determines the first indication Z_{B-1} of the first position zₘₐₓ along the axial direction z in the B-scans 10, which first position zₘₐₓ is the position of the first boundary B-1 of the layer L. This determination is based on an amplitude component of at least one of the B-scans in the sequence of B-scans 10, preferably one of the B-scans 10-1 and 10-2 from which the velocity profile P is generated as described above. Where the layer L is the photoreceptor OS, as in the present case, the first boundary B-1 of the layer L is the IS/OS junction, and the second boundary B-2 of the layer L is the COST, each of which is associated with a respective reflectance peak or maximum in the amplitude of the OCT signal in the B-scan of the sequence of B-scans 10 recorded by the FD-OCT imaging system 30. The data processing apparatus 100 may determine this amplitude peak in a predefined region of the B-scan expected to contain the IS/OS junction and the COST, using any image appropriate image processing technique known to those skilled in the art, for example by finding a peak in a moving average of OCT signal amplitude values, taken along the axial direction in the B-scan, of one or more A-scans of the B-scan. The data processing apparatus 100 is further arranged to determine the second indication Z_{B-2} (of the position along the axial direction z in the B-scan of the IS/OS junction of the OS) by identifying the second position zₘᵢₙ in the velocity profile P corresponding to the minimum velocity vₘᵢₙ in the portion of the velocity profile P. The data processing apparatus 100 may identify the second position zₘᵢₙ in the velocity profile P by firstly calculating, for each combination of a position, zₚₑₐₖ, in the velocity profile P corresponding to that of the determined amplitude peak, and a candidate position, zᵢ, in the velocity profile P of all combinations (zₚₑₐₖ, zᵢ) of zₚₑₐₖ and candidate positions, a respective value of a difference, D_{peak_i}, between a respective velocity vₚₑₐₖ at zₚₑₐₖ and a respective velocity vᵢ at the candidate position zᵢ in the combination (zₚₑₐₖ, zᵢ). The data processing apparatus 100 then identifies, as the second position zₘᵢₙ, a candidate position zᵢ of a combination in the plurality of combinations for which the calculated value of the difference D_{peak_i} is greatest among the calculated values of the difference.

In a further variant of the example embodiment having the functionality described above, the data processing apparatus 100 determines the second indication Z_{B-2} of the second position zₘᵢₙ along the axial direction z in the B-scans 10, which second position zₘᵢₙ is the position of the second boundary B-2 of the layer L. This determination is based on an amplitude component of at least one of the B-scans in the sequence of B-scans 10, preferably one of the B-scans 10-1 and 10-2 from which the velocity profile P is generated as described above. Where the layer L is the photoreceptor OS, as in the present case, the second boundary B-2 of the layer L is the COST, and the first boundary B-1 of the layer L is the IS/OS junction, each of which is associated with a respective reflectance peak or maximum in the amplitude of the OCT signal in the B-scan of the sequence of B-scans 10 recorded by the FD-OCT imaging system 30. The data processing apparatus 100 may determine this amplitude peak in a predefined region of the B-scan expected to contain the IS/OS junction and the COST, using any image appropriate image processing technique known to those skilled in the art, for example by finding a peak in a moving average of OCT signal amplitude values, taken along the axial direction in the B-scan, of one or more A-scans of the B-scan. The data processing apparatus 100 is then further arranged to determine the first indication Z_{B-1} (of the position along the axial direction z in the B-scan of the COST of the OS) by identifying the first position zₘₐₓ in the velocity profile P corresponding to the maximum velocity vₘₐₓ in the portion of the velocity profile P. The data processing apparatus may identify the first position zₘₐₓ in the velocity profile P by firstly calculating, for each combination of a position, zₚₑₐₖ, in the velocity profile P corresponding to that of the determined amplitude peak, and a candidate position, zᵢ, in the velocity profile P of all combinations (zₚₑₐₖ, zᵢ) of zₚₑₐₖ and candidate positions, a respective value of a difference, D_{peak_i}, between a respective velocity vₚₑₐₖ at zₚₑₐₖ and a respective velocity vᵢ at the candidate position zᵢ in the combination (zₚₑₐₖ, zᵢ). The data processing apparatus 100 then identifies, as the first position zₘₐₓ, a candidate position zᵢ of a combination in the plurality of combinations for which the calculated value of the difference D_{peak_i} is greatest among the calculated values of the difference.

The data processing apparatus 100 of the present example embodiment or any of the variants thereof described above may be further arranged to determine whether the first indication Z_{B-1} and/or the second indication Z_{B-2} (as the case may be) determined by the data processing apparatus 100 is reliable or unreliable, and generate an indicator indicative of the determined reliability/unreliability. The reliability of the velocity-based OS segmentation that is performed by the data processing apparatus 100 will be influenced by the image quality of the first B-scan 10-1 and the second B-scan 10-2 (although to a lesser degree than many amplitude-based approaches to segmentation), and how closely correlated the B-scans are to each other. The maximum value of a calculated cross-correlation between the two B-scans provides a good metric of how closely correlated the B-scans are, and therefore the reliability of the first indication Z_{B-1} and/or the second indication Z_{B-2} which is/are determined by the data processing apparatus 100. The data processing apparatus 100 may communicate the determined indicator of reliability to a user (e.g. by displaying a message or other kind of graphic on a screen that informs the user of the determined reliability/unreliability). Additionally or alternatively, the data processing apparatus 100 may store the indicator in association with the first indication Z_{B-1} and/or the second indication Z_{B-2} (as the case may be) which the data processing apparatus 100 has determined. The stored indicator may be used in subsequent data processing operations, as described below.

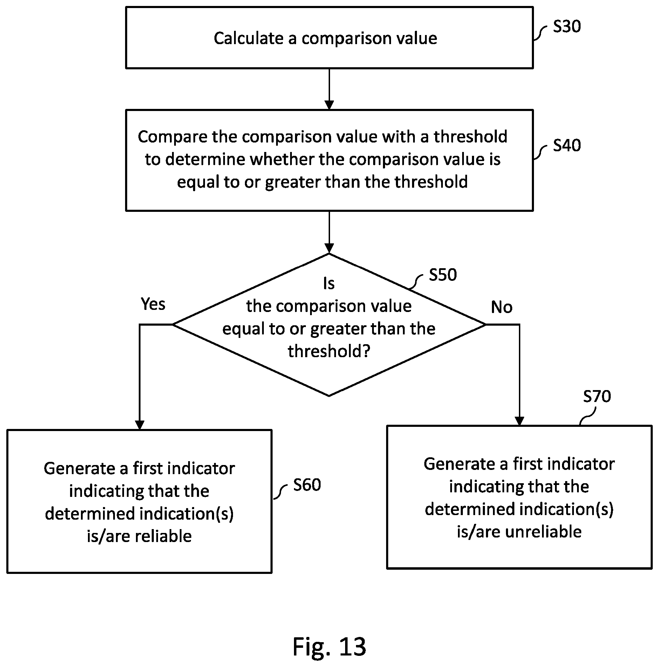

More particularly, the data processing apparatus 100 of the present example embodiment or any of the variants thereof described above may be further arranged to perform the process shown schematically in Figure 13.

In process S30 of Figure 13, the data processing apparatus 100 calculates a comparison value based on based on the first B-scan 10-1 and the second B-scan 10-2. The comparison value may be a value of an image quality metric or attribute calculated based on at least one of an amplitude component of the first B-scan 10-1 and an amplitude component of the second B-scan 10-2. The image quality metric (attribute) may take the form of a dynamic range, a contrast, a signal-to-noise ratio or a sharpness function value from a B-scan. A measure of the success of an intensity-based segmentation of a B-scan is another example of an image quality metric that may be used. Alternatively, the comparison value may be a value of an image similarity metric that provides a measure of a degree of similarity between images, the value of the image similarity metric being calculated based on the first B-scan 10-1 and the second B-scan 10-2. The image similarity metric may, as in the present example embodiment, take the form of a maximum value of the cross-correlation between the first B-scan 10-1 and the second B-scan 10-2. Other image similarity metrics that could alternatively be used include the sum of squared differences, mutual information, normalised mutual information, and Kullback Leibler distance, among others.

The cross-correlation between two complex functions *f*(*t*) and *g*(*t*) of a real variable *t*, denoted *f*g*, is defined by *f*g =f̅*(*t*) * *g*(*t*), where * denotes convolution, and *f̅*(*t*) is the complex conjugate of*f*(*t*).

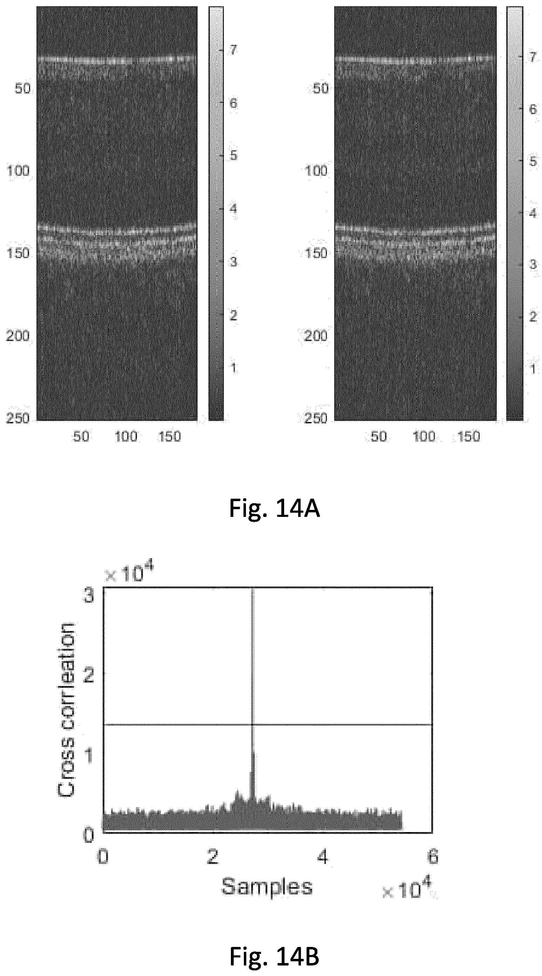

The maximum value of the cross-correlation between two B-scans is a good metric of how closely related the B-scans are to each other. For example, the two B-scans shown in Figure 14A are highly correlated to one another, and the calculated cross-correlation between these B-scans has a high peak value of about 3·10⁴, as shown in Figure 14B.

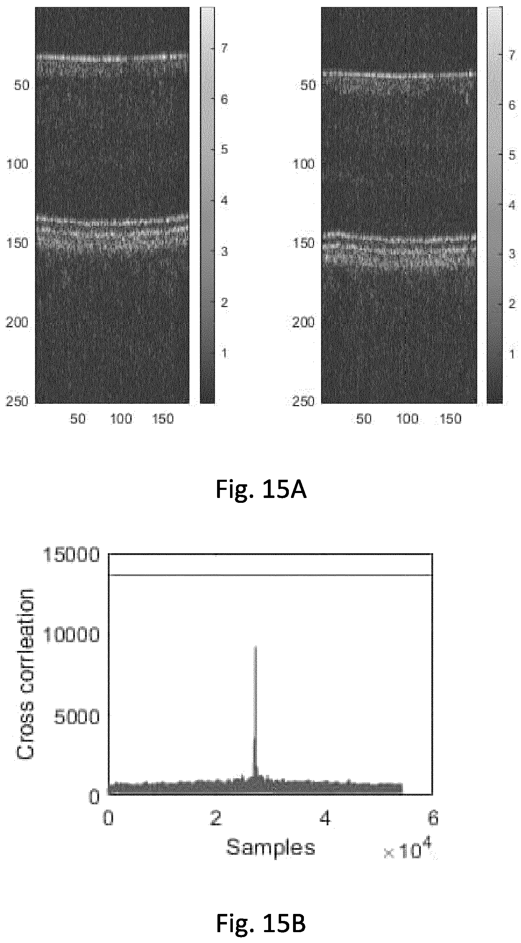

For comparison, Figure 15A shows two B-scans that are dissimilar. This dissimilarity may be caused by factors such as eye movement, for example, and would lead to bad image registration. For the example B-scans shown in Figure 15A, the maximum value of the calculated cross-correlation between the B-scans is much lower than for the B-scans shown in Figure 14A, and is about 1·10⁴, as shown in Figure 15B.

Referring again to Figure 13, in process S40, the data processing apparatus 100 compares the comparison value with a threshold to determine whether the comparison value is equal to or greater than the threshold. The threshold may be set by comparing the segmentation results from the data processing apparatus 100 (i.e. the boundary or boundaries of the layer L indicated by the values of Z_{B-1} and/or Z_{B-2} (as the case may be) determined by the data processing apparatus 100) with a layer segmentation that results from the user's inspection of the source B-scans, whilst having regard to maximum cross-correlation values calculated for the source B-scans. In this way, it may be determined that pairs of B-scans, for which the maximum value of a cross-correlation calculated therebetween is below a certain threshold, tend to yield unreliable values for Z_{B-1} and/or Z_{B-2} (as the case may be) when the B-scans are processed by the data processing apparatus 100 as described herein, whereas pairs of B-scans, for which the maximum value of a cross-correlation calculated therebetween is at or above that threshold, tend to yield reliable values for Z_{B-1} and/or Z_{B-2} (as the case may be).

For example, it may be found that the B-scans shown in Figure 14A yield values of Z_{B-1} and/or Z_{B-2} (as the case may be) that compare favourably with the results of a manual segmentation of the OS performed by inspection of these B-scans, whereas the B-scans shown in Figure 15A yield values of Z_{B-1} and/or Z_{B-2} (as the case may be) that differ significantly from the results of a manual segmentation of the OS performed by inspection of these B-scans. In this case, it may be appropriate to set the threshold to a value between the maximum values of the cross-correlation shown in Figures 5B and 6B (i.e. about 3·10⁴ and 1·10⁴, respectively), for example to a value of about 1.4·10⁴, as shown by the horizontal black line in Figure 15B. B-scans that yield a maximum cross-correlation value below this threshold may be regarded as incapable of allowing reliable segmentation to be performed.

In a case where the comparison value is determined in process S40 to be equal to or greater than the threshold ("Yes" at S50), the data processing apparatus 100 generates a first indicator in process S60 of Figure 13, which indicates that the first indication Z_{B-1} and/or second indication Z_{B-2} determined in process S20 of Figure 3 is reliable.

In a case where the comparison value is determined in process S40 not to be equal to or greater than the threshold ("No" at S50), the data processing apparatus 100 generates a second indicator in process S70 of Figure 13, which indicates that the first indication Z_{B-1} and/or the second indication Z_{B-2} determined in process S20 of Figure 3 is unreliable.

### [Second Example Embodiment]

In the foregoing, a single pair of B-scans, comprising the first B-scan 10-1 and the second B-scan 10-2, is processed by the data processing apparatus 100 to determine the first indication Z_{B-1} and/or the second indication Z_{B-2} and, in some cases, also the first indicator indicating that the determination is reliable or the second indicator indicating that the determination is unreliable. However, the described data processing operations may be used to process multiple pairs of B-scans as in the present example embodiment or C-scans or, more generally, multiple sets of two or more B-scans or C-scans.

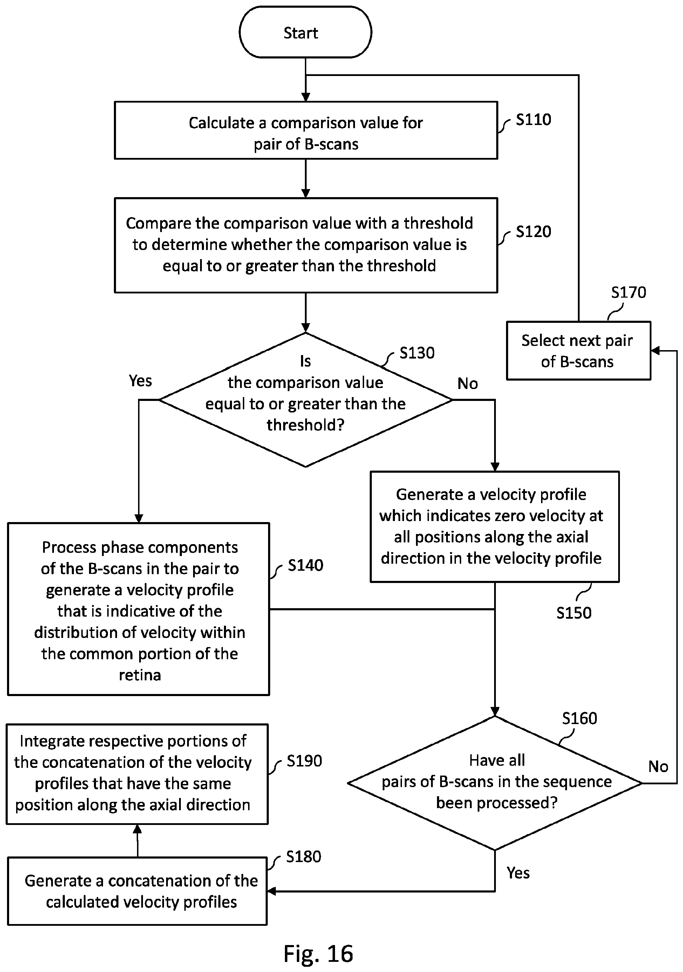

Figure 16 is a flow diagram illustrating a process by which the data processing apparatus 100 of the present example embodiment processes adjacent pairs of adjacent B-scans 10-1 and 10-2 in the sequence of B-scans 10, in turn (e.g. B-scans 10-1 and 10-2, followed by B-scans 10-3 and 10-4, etc. in the sequence of B-scans 10 shown in Figure 1), to generate ORG data that is indicative of the response of the common portion of the retina to an applied light stimulus.

Optoretinography is concerned with minute changes in retinal layers. A registration process of B-scans is often used to account for eye movement that otherwise would engulf an ORG response. Registration comprises shifting two scans spatially relative to each other so that they lie in the best position of similarity defined by the maximum value of the cross-correlation function defined above. The maximum value of this cross-correlation function provides a good indication of how closely matched the two B-scans are. If they are not closely matched, these scans may lead to unrepresentative results, influencing the final ORG outcome. B-scans with poor image quality can also have an adverse effect on the ORG outcome. It may therefore be beneficial to avoid inclusion of velocity profiles from such B-scans is the analysis, and will now be explained.

In process S110 of Figure 16, the data processing apparatus 100 calculates a respective comparison value for each pair of a plurality of different pairs of consecutive B-scans 10-1, 10-2 of the sequence of B-scans 10. Each comparison value may be a value of an image quality metric calculated based on an amplitude component of at least one of the B-scans in the pair. The image quality metric (attribute) may take the form of a dynamic range, a contrast, a signal-to-noise ratio or a sharpness function value from a B-scan, for example. A measure of the success of an intensity-based segmentation of a B-scan is another example of an image quality metric that may be used. Alternatively each comparison value may be a value of an image similarity metric that provides a measure of a degree of similarity between images, the value of the image quality metric being calculated based on amplitude components of the B-scans in the pair. The image similarity metric may take the form of a maximum value of the cross-correlation between the first B-scan 10-1 of the pair and the second B-scan 10-2 of the pair, for example. Other image similarity metrics that could alternatively be used include the sum of squared differences, mutual information, normalised mutual information, and Kullback Leibler distance, among others.

In process S120 of Figure 16, the data processing apparatus 100 compares each comparison value with a threshold to determine whether the comparison value is equal to or greater than the threshold. In the present example embodiment, some pairs of B-scans yield comparison values that are greater than or equal to the threshold, and some other pairs of B-scans yield comparison values that are smaller than the threshold.

Where the calculated comparison value is determined in process S120 of Figure 16 to be equal to or greater than the threshold ("Yes" at S130 of Figure 16), the data processing apparatus 100 processes phase components of the pair of B-scans 10-1 and 10-2, at S140 of Figure 16, as described above in relation to process S10 of Figure 3, to generate a respective velocity profile P that is indicative of the distribution.

On the other hand, where the calculated comparison value is determined in process S120 of Figure 16 not to be greater than or equal to the threshold ("No" at S130 of Figure 16), the data processing apparatus 100 generates, in process S150 of Figure 16, a velocity profile P which indicates zero velocity v at all positions along the axial direction z in the velocity profile P, i.e. a null velocity profile.

Following processes S140 and S150 in Figure 16, the data processing apparatus 100 determines in process S160 whether all pairs of adjacent B-scans in the sequence of B-scans 10 have been processed. If not ("No" at S160), the data processing apparatus 100 selects the next pair of adjacent B-scans in the sequence of B-scans 10 (i.e. a pair of B-scans which is adjacent to the last pair of B-scans having been processed up to that point in the process), and the selected pair of B-scans is then process as described above, starting at process S110 of Figure 16. However, if the data processing apparatus 100 determines in process S160 that all pairs of adjacent B-scans in the sequence of B-scans 10 have already been processed ("Yes" at S160 of Figure 16), the processing proceeds to S180 of Figure 16, where the data processing apparatus 100 concatenates the generated velocity profiles P, such that the concatenation of the velocity profiles is indicative of how the distribution changes over time.

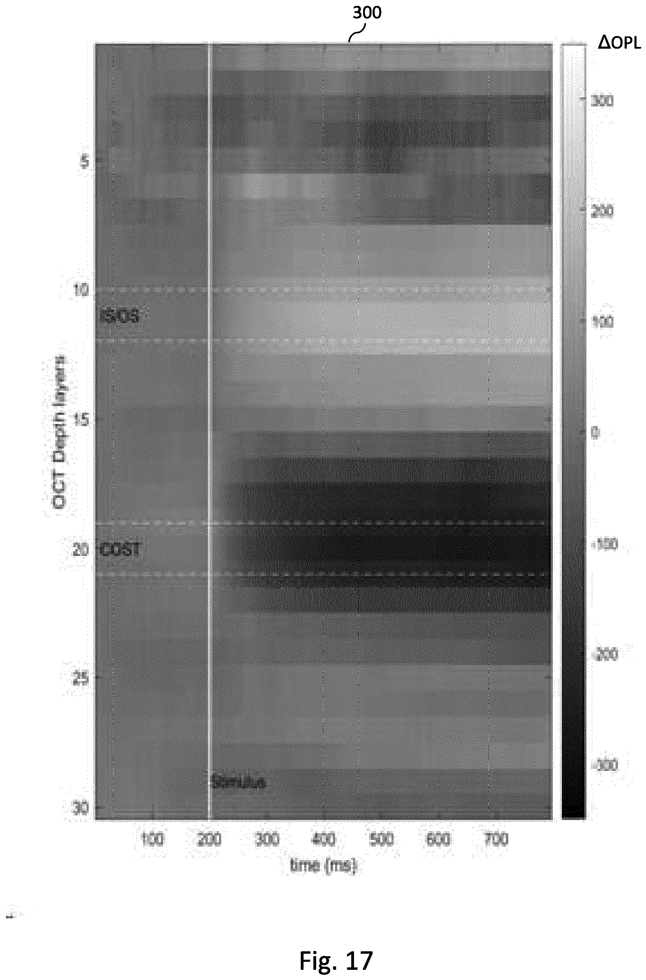

Figure 17 shows an example of a concatenation 300 of the velocity profiles P generated by the data processing apparatus 100 in process S180 of Figure 16. Each velocity profile P extends along the y-axis (labelled "OCT depth layers") in Figure 17, and the velocity profiles calculated for adjacent pairs of B-scans in the sequence of B-scans 10 are arrayed along the x-axis (labelled "time (ms)") in Figure 17. Figure 17 shows the changes in optical path length, ΔOPL, (which provides an indication of the velocity v) with position in each velocity profile P, which is derived from B-scans that have been acquired after application of the optical stimulus at time t = 200 ms. The change in optical path length ΔOPL varies with position in each velocity profile (i.e. along the y-axis in Figure 17) from a minimum ΔOPL of about -300 (arb. unit) to a maximum ΔOPL of about +250 (arb. unit). Although there is some variation among the velocity profiles P in the distribution of ΔOPL with position, particularly among velocity profiles for times between 200 ms and 300 ms (shortly after the application of the light stimulus at time t = 200 ms), each velocity profile nevertheless has a global maximum in the vicinity of OCT depth layer 11, which is associated with the IS/OS, and a global minimum in the vicinity of OCT depth layer 20, which is associated with the COST.

Referring again to Figure 16, in process S190, the data processing apparatus 100 integrates respective portions of velocity profiles P in the concatenation of the velocity profiles, which portions have the same position along the axial direction z, to generate ORG data indicating an optical path length variation over time at the position along the axial direction z. In process S190 of Figure 16, the data processing apparatus 100 may process each velocity profile P in the concatenated set of velocity profiles by selecting a portion (segment or data element) of the velocity profile P that is disposed at a predetermined position along the axial direction z of the velocity profile P, and then integrate the selected (commonly located) portions by calculating a cumulative sum (or running total) of the velocity values in these portions, which indicates how an optical path length of an optical path of an OCT sample beam that ends at a location in the retina corresponding to the predetermined position in the velocity profile P changes over time.

An indication of an optical path length variation over time at the first position along the axial direction z indicated by the first indication Z_{B-1} may be determined by integrating (i.e. calculating a cumulative sum or running total of) respective portions of the concatenation at the first position along the axial direction z. Additionally or alternatively, an indication of an optical path length variation over time at the second position along the axial direction z indicated by the second indication Z_{B-2} may be determined by integrating (i.e. calculating a cumulative sum or running total of) respective portions of the concatenation at the second position along the axial direction z.

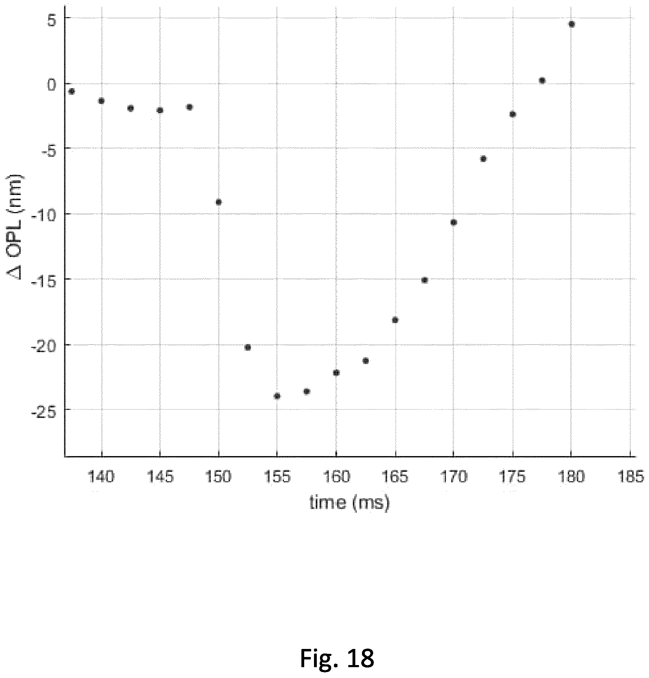

Figure 18 shows a plot of changes in optical path length ΔOPL over time, which have been calculated by the data processing apparatus 100 for a selected position in the velocity profiles of the concatenation of velocity profiles. The plot in Figure 18 shows how ΔOPL at the common position changes from one B-scan to the next in the sequence of B-scans 10.

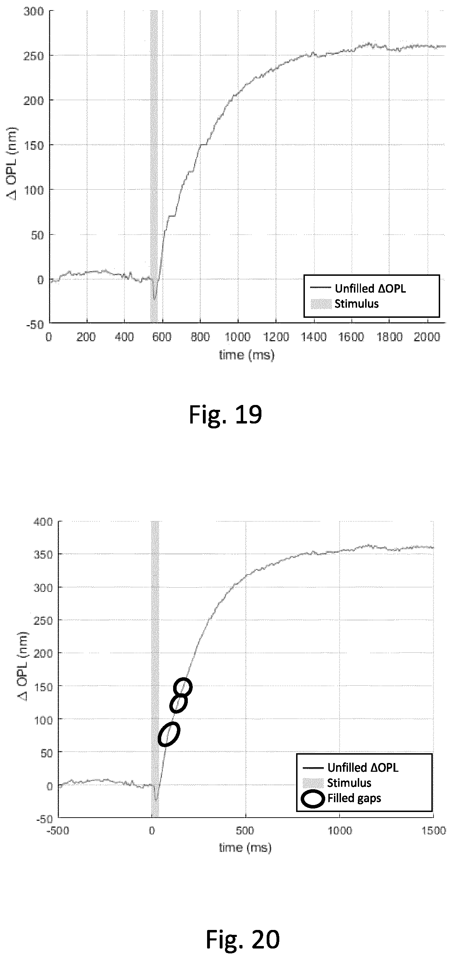

Figure 19 shows a plot of cumulative changes in the optical path length over time, which is known as the ORG signal (or ORG data). The ORG signal is obtained by calculating a cumulative sum of optical path length changes for a selected position in the velocity profiles of the concatenation of velocity profiles. As shown in Figure 19, the ORG signal becomes negative shortly after the stimulus is applied, and then increases to become positive, eventually levelling off at a value of approximately 250 nm in this example. Figure 19 also shows the ORG signal to have several plateaus, which are caused by the replacements of velocity profiles calculated using B-scans that do not yield a sufficiently high maximum cross-correlation value with velocity profiles that indicate zero velocity (i.e. null velocity profiles), as described above.

The ORG data generated in process S190 of Figure 16 may thus comprise one or more sets of equal consecutive values, and the data processing apparatus 100 may be arranged to smooth the generated ORG data by replacing one or more values in at least one set of the one or more sets of equal consecutive values with one or more estimated values calculated based on neighbouring values that neighbour the set of equal consecutive values in the ORG data. The data processing apparatus 100 may perform this smoothing by using a moving median or a moving mean, for example, where a median or mean of a specified number of points either side of the missing data point(s) is calculated and then assigned to the missing point(s). Alternatively, the data processing apparatus 100 may, for example, detect each set of equal consecutive values in the ORG data, and replace the values in each detected set with corresponding estimated values that are obtained by interpolating (e.g. linearly) between a first value in the ORG data which is adjacent to the first of the equal consecutive values in the detected set, and a second value in the ORG data which is adjacent to the last of the equal consecutive values in the detected set.

A result of smoothing the ORG signal using a moving mean is shown in Figure 20. Before the smoothing has been applied, large portions of the data represent OPL changes of zero, as shown in Figure 19, and thus generally underrepresent actual values that would be observed. Using the method described herein, the equal values are replaced with estimated values (as indicated by circles in Figure 20), which represent the change in OPL more accurately.

Although the data processing apparatus 100 processes pairs of adjacent B-scans in the sequence of B-scans 10 in turn (i.e. one pair after another adjacent pair in the sequence) to generate the velocity profiles P, the data processing apparatus 100 may alternatively generate the velocity profiles P by processing pairs of adjacent B-scans in parallel, thereby greatly speeding up the process. Furthermore, although pairs of B-scans that are adjacent to each other in the sequence of B-scans 10 (i.e. consecutive B-scans in the sequence) are processed, the data processing apparatus 100 may alternatively process pairs of B-scans in the sequence where the B-scans of each pair are separated from each other by one or more intervening B-scans, for example.

Further still, although the data processing apparatus 100 of the present example embodiment has been described as processing pairs of B-scans, it may more generally be configured to process sets of more than two B-scans in the sequence of B-scans 10, which B-scans may be consecutive B-scans in the sequence or individual B-scans in the sequence that are separated from each other by one or more intervening B-scans not forming part of the set. The respective sets of (e.g. 5) B-scans used in the loops of the process of Figure 16 may be selected by sliding a window selection function along the sequence of B-scans 10, where, in each loop of the process, the window selection function selects all B-scans in a windowed portion of the sequence of B-scans 10 or every other B-scan in the windowed portion, for example, and the selected sets may have one or more B-scans in common. In such other example embodiments, a modified version of the process described above with reference to Figure 16, which is based on the technique described in Kari V. Vienola, et al., "Velocity-based optoretinography for clinical applications," Optica 9, 1100-1108 (2022), would be as follows.

In the modified form of process S110 in Figure 16, the data processing apparatus 100 calculates a comparison value for a set of a plurality of different sets of consecutive B-scans in the sequence of B-scans 10. The comparison value may take any of the different forms described above. Then, as in process S120 of Figure 16, the data processing apparatus 100 compares the comparison value with a threshold to determine whether the comparison value is equal to or greater than the threshold. Some sets of B-scans yield comparison values that are greater than or equal to the threshold, and some other sets of B-scans yield comparison values that are smaller than the threshold. Where the calculated comparison value is determined to be equal to or greater than the threshold, the data processing apparatus 100 processes phase components of at least some of the B-scans in the set in accordance with a modified form of process S140 of Figure 16, to generate a velocity profile P that is indicative of the distribution. Here, the data processing apparatus 100 may first flatten the B-scans of the set such that the IS/OS and COST reflections lie at substantially the same height for each A-scan in each of the B-scans. The data processing apparatus 100 may then register the B-scans with respect to each other. The phase data cube *θ*(*x, z, t*) is then unwrapped in the temporal dimension by adding or subtracting 2π to θ(*x*ₚ, *z*_{q}, *t*ᵣ) to minimise |θ(*xₚ, z_{q}, tᵣ*) *-* θ(*xₚ, z_{q}, tᵣ₋₁*)|, where *tᵣ* and *tᵣ₋₁* represent consecutive phase B-scans. This step is performed for each spatial coordinate pair (*xₚ, z_{q}*) in the volume. A rate of phase change is computed for each coordinate pair by performing a least-squares linear fit with respect to *t*, giving $\frac{Δ θ}{Δ t} \left(x, z\right)$ in rad/s. From this, the instantaneous velocity for each spatial location may be calculated as $\frac{Δ z}{Δ t} \left(x, z\right) = \frac{Δ θ}{Δ t} \left(x, z\right) ⋅ \frac{λ}{4 πn}$, where λ is the wavelength of OCT light being used, and *n* is a nominal refractive index of the eye 20. These instantaneous velocities may be averaged in the lateral dimension (i.e. along the x-axis) to give instantaneous, depth-dependent measures of velocity along the z-axis, i.e. a one-dimensional velocity profile P of the kind illustrated schematically in Figure 1, which is indicative of a distribution of velocity v along the axial direction (z-axis) within the common portion of the retina that is covered by the repeat B-scans. The B-scan amplitudes (if desired) can also be averaged in the lateral dimension (x-axis) to give instantaneous, depth-dependent measures of backscattering. It should be noted that averaging in the lateral dimension is not required, and a two-dimensional velocity profile may alternatively be generated, which is indicative of the distribution of velocity v along both the axial direction (z-axis) and the lateral direction (x-axis) within the common portion of the retina that is covered by the repeat B-scans. On the other hand, where the calculated comparison value is determined not to be equal to or greater than the threshold, the data processing apparatus 100 generates, as in process S150 of Figure 16, a velocity profile P which indicates zero velocity v at all positions along the axial direction z in the velocity profile P, i.e. a null velocity profile. The data processing apparatus 100 of the other examiner embodiments mentioned above would perform the further processes as described above with reference to Figure 16, although with references to "pairs" of B-scans in processes S160 and 170 of Figure 16 being replaced with the "sets" of B-scans discussed above.

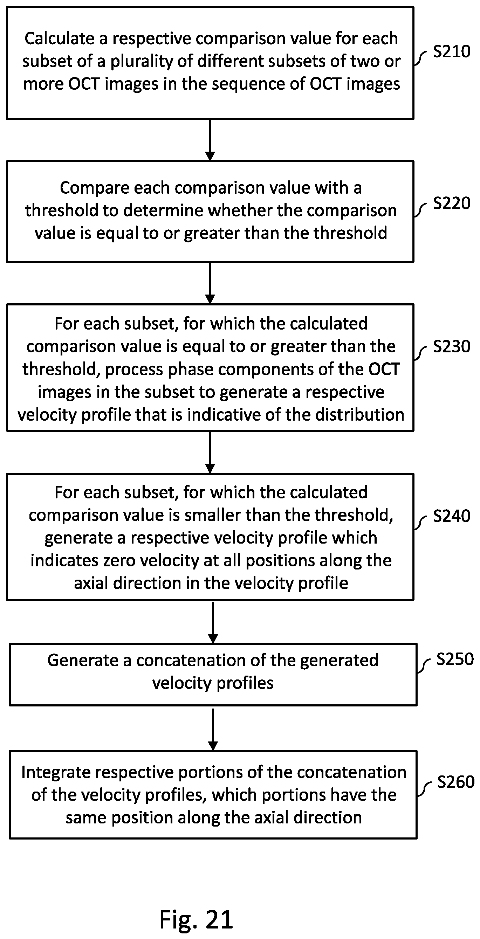

The data processing apparatus 100 may more generally be arranged to perform a process which will now be described with reference to Figure 21.

In process S210 of Figure 21, the data processing apparatus 100 calculates a respective comparison value for each set of a plurality of different sets of two or more OCT images in the sequence of OCT images 10. The respective comparison value may be a respective value of an image quality metric calculated based on an amplitude component of at least one of the OCT images in the set, or a respective value of an image similarity metric that provides a measure of a degree of similarity between images, the value of the image quality metric being calculated based on amplitude components of at least two of the OCT images in the set. The comparison value may take any of the example forms described above.

In process S220 of Figure 21, the data processing apparatus 100 compares each comparison value with a threshold to determine whether the comparison value is equal to or greater than the threshold.

For each set of the plurality of different sets of OCT images, for which set the calculated comparison value is determined to be equal to or greater than the threshold, the data processing apparatus 100 processes, in process S230 of Figure 21, phase components of the OCT images in the set to generate a respective velocity profile that is indicative of the distribution.

For each set of the plurality of different sets of OCT images, for which set the calculated comparison value is determined not to be greater than or equal to the threshold, the data processing apparatus 100 generates, in process S240 of Figure 21, a respective velocity profile P which indicates zero velocity v at all positions along the axial direction z in the velocity profile P.

In process S250 of Figure 21, the data processing apparatus 100 generates a concatenation of the generated velocity profiles such that the concatenation of the velocity profiles is indicative of how the distribution changes over time.

Finally, in process S260 of Figure 21, the data processing apparatus 100 integrates respective portions of the concatenation of the velocity profiles, which portions have the same position along the axial direction, to generate data indicating an optical path length variation over time at the position along the axial direction.

### [Third Example Embodiment]

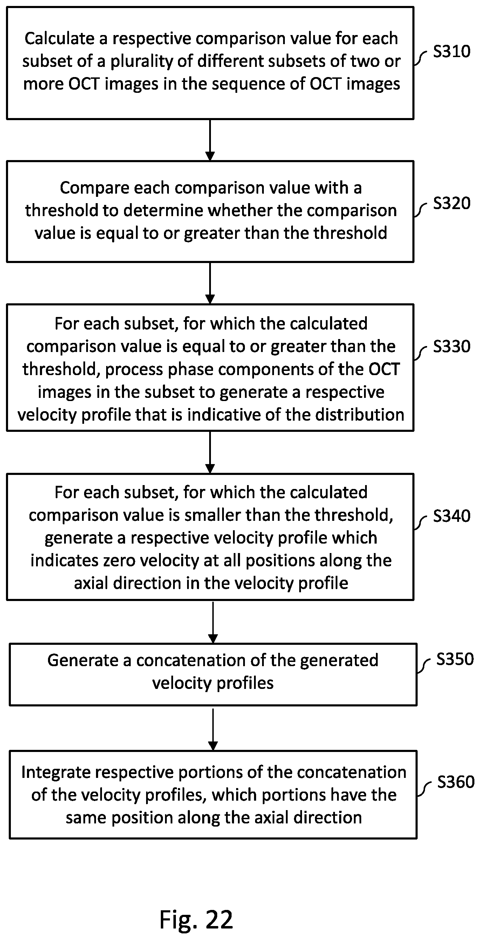

In the second example embodiment described above, the data processing apparatus 100 may reduce a degradation in the quality of the generated ORG data by using comparison values calculated for sets of two or more B-scans in the sequence of B-scans 10 to set, for use in the generation of the ORG data and in place of any velocity profile derived from a set in which one or more of the constituent B-scans has unacceptable image quality or in which two or more constituent B-scans have insufficient degree of similarity, a velocity profile indicating zero velocity throughout (i.e. a null velocity profile), and to optionally suppress any resulting artefacts in the ORG data by smoothing the ORG data. However, the data processing apparatus 100 may, as in the present example embodiment, alternatively be arranged to reduce the degradation in quality of the generated ORG data by replacing any velocity profile, which is derived from a set in which one or more of the constituent B-scans has unacceptable image quality or in which two or more constituent B-scans have insufficient degree of similarity, with an estimated (rather than a null) velocity profile, as will now be described in more detail with reference to Figure 22.

Processes S310 to S330, S350 and S360 of Figure 22 are the same as processes S210 to S230, S250 and S260 of Figure 21, respectively, which have been described in detail above.

In process S340 of Figure 22, the data processing apparatus 100 generates, for each set of the plurality of different sets of B-scans, for which set the calculated comparison value is determined not to be greater than or equal to the threshold (i.e. smaller than the threshold), a respective estimated velocity profile P that is indicative of the distribution of velocity v along the axial direction (z-axis) within the common portion of the retina that is covered by the B-scans. Each estimated velocity profile is based on respective one or more velocity profiles that have each been calculated in accordance with process S330 of Figure 22, and is/are adjacent the estimated velocity profile in the concatenation of velocity profiles that is generated in process S350 in Figure 22. In other words, the data processing apparatus 100 of the present example embodiment generates, for each unreliable set of two or more B-scans, for which the calculated comparison value is determined not to be greater than or equal to the threshold, a respective velocity profile P based on an adjacent reliable set of two or more B-scans (for which the calculated comparison value is determined to be equal to or greater than the threshold) instead of the given set of two or more B-scans. In the present example embodiment, the ORG data generated in process S360 of Figure 22 does not have plateaus as shown in Figure 19, and smoothing of the ORG data may therefore not be necessary.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the processing methods described with reference to Figures 3, 13, 16, 21 and 22, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the OCT data processing hardware 130 may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable subcombination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. A computer-implemented method of processing a phase component of a first OCT image (10-1) and a phase component of a second OCT image (10-2) of a sequence of OCT images (10) of a common portion of a retina of an eye (20) acquired by a Fourier-domain optical coherence tomography, OCT, imaging system (30) after stimulation of the common portion by an optical stimulus (40), wherein the common portion comprises a layer (L) of the retina whose thickness changed in response to the optical stimulus (40) during acquisition of the sequence of OCT images (10), to determine at least one of a first indication (Z_{B-1}) of a first position along an axial direction (z) in the OCT images (10) of a first boundary (B-1) of the layer (L) and a second indication (Z_{B-2}) of a second position along the axial direction (z) in the OCT images (10) of a second boundary (B-2) of the layer (L), the method comprising:
processing (S10) the phase component of the first OCT image (10-1) and the phase component of the second OCT image (10-2) to calculate a velocity profile (P) indicative of a distribution, along the axial direction (z), of velocity (v) within the common portion of the retina; and
determining (S20) the at least one of the first indication (Z_{B-1}) and the second indication (Z_{B-2}) based on the calculated velocity profile (P),
wherein the at least one of the first indication (Z_{B-1}) and the second indication (Z_{B-2}) is determined based on the calculated velocity profile (P) by:
determining, where the first indication (Z_{B-1}) is determined, a first position in the velocity profile (P) corresponding to a maximum of velocity (v) indicated by the velocity profile(P) as the first indication (Z_{B-1}); and
determining, where the second indication (Z_{B-2}) is determined, a second position in the velocity profile (P) corresponding to a minimum of velocity (v) indicated by the velocity profile (P) as the second indication (Z_{B-2}).

2. The computer-implemented method according to Claim 1, wherein the at least one of the first indication (Z_{B-1}) and the second indication (Z_{B-2}) is determined by processing the calculated velocity profile (P) using a cluster analysis algorithm or a dimensionality reduction algorithm.

3. The computer-implemented method according to Claim 2, wherein the dimensionality reduction algorithm comprises one of a principal component analysis algorithm, an independent component analysis algorithm, a linear discriminant analysis algorithm or a non-negative matrix factorisation algorithm.

4. The computer-implemented method according to Claim 3, wherein the dimensionality reduction algorithm comprises a principal component analysis algorithm, and the at least one of the first indication (Z_{B-1}) and the second indication (Z_{B-2}) is determined using principal components that have been determined by applying the principal component analysis algorithm to the calculated velocity profile (P).

5. The computer-implemented method according to Claim 1, wherein both the first position in the velocity profile (P) and the second position in the velocity profile (P) are determined by:
calculating, for each combination of a candidate first position in the velocity profile (P) and a candidate second position in the velocity profile (P) of all combinations of candidate first positions and candidate second positions in the velocity profile (P), a respective value of a difference between a respective velocity (v) indicated by the velocity profile (P) for the candidate first position and a respective velocity (v) indicated by the velocity profile (P) for the candidate second position in the combination; and
identifying, as the first position in the velocity profile (P) and the second position in the velocity profile (P), a candidate first position and a candidate second position of a combination in the plurality of combinations for which the calculated value of the difference is greatest among the calculated values of the difference.

6. The computer-implemented method according to Claim 1, wherein both the first position in the velocity profile (P) and the second position in the velocity profile (P) are determined by:
calculating, for each combination of a candidate first position in the velocity profile (P) and a candidate second position in the velocity profile of all combinations of candidate first positions and candidate second positions in the velocity profile (P), a respective value of a difference between an average of respective velocities indicated by the velocity profile (P) for a set of adjacent positions including the candidate first position in the combination, and an average of respective velocities indicated by the velocity profile (P) for a set of adjacent positions including the candidate second position in the combination; and
identifying, as the first position in the velocity profile and the second position in the velocity profile (P), the candidate first position and the candidate second position of a combination of the plurality of combinations for which the calculated value of the difference is greatest among the calculated values of the difference.

7. The computer-implemented method according to any of Claims 1 to 6, further comprising one of:
determining the first indication (Z_{B-1}) of the first position along the axial direction (z) in the OCT images (10) of the first boundary (B-1) of the layer (L) based on an amplitude component of at least one of the OCT images in the sequence of OCT images (10), wherein the second indication (Z_{B-2}) is determined by identifying the second position in the velocity profile (P) corresponding to a minimum of velocity indicated by the velocity profile (P); and
determining the second indication (Z_{B-2}) of the second position along the axial (z) direction in the OCT images (10) of the second boundary (B-2) of the layer (L) based on an amplitude component of at least one of the OCT images in the sequence of OCT images (10), wherein the first indication (Z_{B-1}) is determined by identifying the first position in the velocity profile (P) corresponding to a maximum velocity indicated by the velocity profile (P).

8. The computer-implemented method according to any of Claims 1 to 7, further comprising:
calculating a comparison value being one of:
a value of an image quality metric calculated based on at least one of an amplitude component of the first OCT image (10-1) and an amplitude component of the second OCT image (10-2); and
a value of an image similarity metric that provides a measure of a degree of similarity between images, the value of the image similarity metric being calculated based on the amplitude component of the first OCT image (10-1) and the amplitude component of the second OCT image (10-2);
comparing the comparison value with a threshold to determine whether the comparison value is equal to or greater than the threshold;
in a case where the comparison value is determined to be equal to or greater than the threshold, generating a first indicator indicating that the determined the at least one of the first indication (Z_{B-1}) and the second indication (Z_{B-2}) is reliable; and
in a case where the comparison value is determined not to be equal to or greater than the threshold, generating a second indicator indicating that the determined the at least one of the first indication (Z_{B-1}) and the second indication (Z_{B-2}) is unreliable.

9. The computer-implemented method according to Claim 8, wherein the comparison value is a maximum value of a calculated cross-correlation between the first OCT image (10-1) and the second OCT image (10-2).

10. The computer-implemented method according to any of Claims 1 to 7, further comprising:
calculating, for each set of a plurality of different sets of two or more OCT images (10-1, 10-2) in the sequence of OCT images (10), a respective comparison value being one of:
a respective value of an image quality metric calculated based on an amplitude component of at least one of the OCT images in the set; and
a respective value of an image similarity metric that provides a measure of a degree of similarity between images, the value of the image quality metric being calculated based on amplitude components of at least two of the OCT images in the set;
comparing each comparison value with a threshold to determine whether the comparison value is equal to or greater than the threshold;
for each set of the plurality of different sets of OCT images, for which set the calculated comparison value is determined to be equal to or greater than the threshold, processing phase components of the OCT images in the set to generate a respective velocity profile that is indicative of the distribution;
for each set of the plurality of different sets of OCT images, for which set the calculated comparison value is determined not to be greater than or equal to the threshold, generating a respective velocity profile (P) which indicates zero velocity (v) at all positions along the axial direction (z) in the velocity profile (P);
generating a concatenation (300) of the generated velocity profiles such that the concatenation (300) of the velocity profiles is indicative of how the distribution changes over time; and
integrating respective portions of the concatenation (300) of the velocity profiles, which portions have the same position along the axial direction, to generate data indicating an optical path length variation over time at the position along the axial direction.

11. The computer-implemented method according to Claim 10, wherein
the generated data comprises one or more sets of equal consecutive values, and
the method further comprises smoothing the generated data by replacing one or more values in a set of the one or more sets of equal consecutive values with one or more estimated values calculated based on neighbouring values that neighbour the set of equal consecutive values in the generated data.

12. The computer-implemented method according to any of Claims 1 to 7, further comprising:
calculating, for each set of a plurality of different sets of two or more OCT images (10-1, 10-2) in the sequence of OCT images (10), a respective comparison value being one of:
a respective value of an image quality metric calculated based on an amplitude component of at least one of the OCT images in the set; and
a respective value of an image similarity metric that provides a measure of a degree of similarity between images, the value of the image quality metric being calculated based on amplitude components of at least two of the OCT images in the set;
comparing each comparison value with a threshold to determine whether the comparison value is equal to or greater than the threshold;
for each set of the plurality of different sets of OCT images, for which set the calculated comparison value is determined to be equal to or greater than the threshold, processing phase components of the OCT images in the set to generate a respective velocity profile that is indicative of the distribution;
for each set of the plurality of different sets of OCT images, for which set the calculated comparison value is determined not to be greater than or equal to the threshold, generating a respective velocity profile that is indicative of the distribution, based on a respective velocity profile calculated for each set of one or more other sets of the plurality of different sets of OCT images;
generating a concatenation (300) of the generated velocity profiles such that the concatenation (300) of the velocity profiles is indicative of how the distribution changes over time; and
integrating respective portions of the concatenation (300) of the velocity profiles, which portions have the same position along the axial direction, to generate data indicating an optical path length variation over time at the position along the axial direction.

13. The computer-implemented method according to any of Claims 10 to 12, wherein the respective comparison value calculated for each set is a respective maximum value of a calculated cross-correlation between at least two of the OCT images in the set.

14. The computer-implemented method according to any of Claims 10 to 13, wherein a respective indication of an optical path length variation over time at each of at least one of the first position along the axial direction (z) and the second position along the axial direction (z) indicated by the at least one of the first indication (Z_{B-1}) and the second indication (Z_{B-2}) is determined by integrating respective portions of the concatenation (300) at the at least one of the first position along the axial direction (z) and the second position along the axial direction (z).

15. The computer-implemented method according to any of Claims 1 to 14, wherein the layer of the retina comprises an outer segment of photoreceptor cells.

16. The computer-implemented method according to any of Claims 1 to 15, further comprising processing the phase component of the first OCT image (10-1) and the phase component of the second OCT image (10-2), before calculation of the velocity profile (P), to compensate for a bulk motion of the common portion of the retina during acquisition of the sequence of OCT images (10) by a Fourier-domain OCT imaging system (30) after stimulation of the common portion by the optical stimulus (40).

17. A computer program (245) comprising computer-readable instructions which, when executed by a processor (220), cause the processor (200) to perform a method according to any of Claims 1 to 16.

18. A data processing apparatus (100) arranged to process a phase component of a first OCT image (10-1) and a phase component of a second OCT image (10-2) of a sequence of OCT images (10) of a common portion of a retina of an eye (20) acquired by a Fourier-domain optical coherence tomography, OCT, imaging system (30) after stimulation of the common portion by an optical stimulus (40), wherein the common portion comprises a layer (L) of the retina whose thickness changed in response to the optical stimulus (40) during acquisition of the sequence of OCT images (10), to determine at least one of a first indication (Z_{B-1}) of a first position along an axial direction (z) in the OCT images (10) of a first boundary (B-1) of the layer (L) and a second indication (Z_{B-2}) of a second position along the axial direction (z) in the OCT images (10) of a second boundary (B-2) of the layer (L), the data processing apparatus (100) being arranged to:
process (S10) the phase component of the first OCT image (10-1) and the phase component of the second OCT image (10-2) to calculate a velocity profile (P) indicative of a distribution, along the axial direction (z), of velocity (v) within the common portion of the retina; and
determine (S20) the at least one of the first indication (Z_{B-1}) and the second indication (Z_{B-2}) based on the calculated velocity profile (P),
wherein the data processing apparatus (100) is arranged to determine the at least one of the first indication (Z_{B-1}) and the second indication (Z_{B-2}) based on the calculated velocity profile by:
determining, where the first indication (Z_{B-1}) is determined, a first position (zₘₐₓ) in the velocity profile (P) corresponding to a maximum of velocity (vₘₐₓ) indicated by the velocity profile (P) as the first indication (Z_{B-1}); and
determining, where the second indication (Z_{B-2}) is determined, a second position (zₘᵢₙ) in the velocity profile (P) corresponding to a minimum of velocity (vₘᵢₙ) indicated by the velocity profile (P) as the second indication (Z_{B-2}).

## Patentansprüche

1. Ein computerimplementiertes Verfahren zum Verarbeiten einer Phasenkomponente eines ersten OCT-Bildes (10-1) und einer Phasenkomponente eines zweiten OCT-Bildes (10-2) einer Sequenz von OCT-Bildern (10) eines gemeinsamen Abschnitts einer Retina eines Auges (20), die durch ein Fourier-Domänen-Optische-Kohärenztomographie-, OCT, Bildgebungssystem (30) nach Stimulation des gemeinsamen Abschnitts durch einen optischen Stimulus (40) erfasst wurde, wobei der gemeinsame Abschnitt eine Schicht (L) der Retina umfasst, deren Dicke sich als Reaktion auf den optischen Stimulus (40) während der Erfassung der Sequenz von OCT-Bildern (10) veränderte, um mindestens eine einer ersten Angabe (Z_{B-1}) einer ersten Position entlang einer axialen Richtung (z) in den OCT-Bildern (10) einer ersten Grenze (B-1) der Schicht (L) und einer zweiten Angabe (Z_{B-2}) einer zweiten Position entlang der axialen Richtung (z) in den OCT-Bildern (10) einer zweiten Grenze (B-2) der Schicht (L) zu bestimmen, das Verfahren umfassend:
Verarbeiten (S10) der Phasenkomponente des ersten OCT-Bildes (10-1) und der Phasenkomponente des zweiten OCT-Bildes (10-2), um ein Geschwindigkeitsprofil (P) zu berechnen, das eine Verteilung, entlang der axialen Richtung (z), der Geschwindigkeit (v) innerhalb des gemeinsamen Abschnitts der Retina angibt; und
Bestimmen (S20) der mindestens einen der ersten Angabe (Z_{B-1}) und der zweiten Angabe (Z_{B-2}) basierend auf dem berechneten Geschwindigkeitsprofil (P),
wobei die mindestens eine der ersten Angabe (Z_{B-1}) und der zweiten Angabe (Z_{B-2}) basierend auf dem berechneten Geschwindigkeitsprofil (P) bestimmt wird durch:
Bestimmen, wenn die erste Angabe (Z_{B-1}) bestimmt ist, einer ersten Position in dem Geschwindigkeitsprofil (P), die einem durch das Geschwindigkeitsprofil (P) angegebenen Maximum der Geschwindigkeit (v) entspricht, als die erste Angabe (Z_{B-1}); und
Bestimmen, wenn die zweite Angabe (Z_{B-2}) bestimmt ist, einer zweiten Position in dem Geschwindigkeitsprofil (P), die einem durch das Geschwindigkeitsprofil (P) angegebenen Minimum der Geschwindigkeit (v) entspricht, als die zweite Angabe (Z_{B-2}).

2. Das computerimplementierte Verfahren nach Anspruch 1, wobei die mindestens eine der ersten Angabe (Z_{B-1}) und der zweiten Angabe (Z_{B-2}) durch das Verarbeiten des berechneten Geschwindigkeitsprofils (P) unter Verwendung eines Clusteranalyse-Algorithmus oder eines Algorithmus zur Dimensionsreduktion bestimmt wird.

3. Das computerimplementierte Verfahren nach Anspruch 2, wobei der Algorithmus zur Dimensionsreduktion einen eines Hauptkomponentenanalyse-Algorithmus, eines Algorithmus der unabhängigen Komponentenanalyse, eines Algorithmus der linearen Diskriminanzanalyse oder eines Algorithmus der nichtnegativen Matrixfaktorisierung umfasst.

4. Das computerimplementierte Verfahren nach Anspruch 3, wobei der Algorithmus zur Dimensionsreduktion einen Hauptkomponentenanalyse-Algorithmus umfasst, und die mindestens eine der ersten Angabe (ZB-1) und der zweiten Angabe (ZB-2) unter Verwendung von Hauptkomponenten bestimmt wird, die durch das Anwenden des Hauptkomponentenanalyse-Algorithmus auf das berechnete Geschwindigkeitsprofil (P) bestimmt wurden.

5. Das computerimplementierte Verfahren nach Anspruch 1, wobei sowohl die erste Position in dem Geschwindigkeitsprofil (P) als auch die zweite Position in dem Geschwindigkeitsprofil (P) bestimmt werden durch:
Berechnen, für jede Kombination einer ersten Kandidatenposition in dem Geschwindigkeitsprofil (P) und einer zweiten Kandidatenposition in dem Geschwindigkeitsprofil (P) aus allen Kombinationen von ersten Kandidatenpositionen und zweiten Kandidatenpositionen in dem Geschwindigkeitsprofil (P), eines jeweiligen Wertes einer Differenz zwischen einer jeweiligen Geschwindigkeit (v), die durch das Geschwindigkeitsprofil (P) für die erste Kandidatenposition angegeben wird, und einer jeweiligen Geschwindigkeit (v), die durch das Geschwindigkeitsprofil (P) für die zweite Kandidatenposition in der Kombination angegeben wird; und
Identifizieren, als die erste Position in dem Geschwindigkeitsprofil (P) und die zweite Position in dem Geschwindigkeitsprofil (P), einer ersten Kandidatenposition und einer zweiten Kandidatenposition einer Kombination in der Vielzahl von Kombinationen, für welche der berechnete Wert der Differenz unter den berechneten Werten der Differenz am größten ist.

6. Das computerimplementierte Verfahren nach Anspruch 1, wobei sowohl die erste Position in dem Geschwindigkeitsprofil (P) als auch die zweite Position in dem Geschwindigkeitsprofil (P) bestimmt werden durch:
Berechnen, für jede Kombination einer ersten Kandidatenposition in dem Geschwindigkeitsprofil (P) und einer zweiten Kandidatenposition in dem Geschwindigkeitsprofil aus allen Kombinationen von ersten Kandidatenpositionen und zweiten Kandidatenpositionen in dem Geschwindigkeitsprofil (P), eines jeweiligen Wertes einer Differenz zwischen einem Durchschnitt von jeweiligen Geschwindigkeiten, die durch das Geschwindigkeitsprofil (P) für einen Satz von benachbarten Positionen einschließlich der ersten Kandidatenposition in der Kombination angegeben werden, und einem Durchschnitt von jeweiligen Geschwindigkeiten, die durch das Geschwindigkeitsprofil (P) für einen Satz von benachbarten Positionen einschließlich der zweiten Kandidatenposition in der Kombination angegeben werden; und
Identifizieren, als die erste Position in dem Geschwindigkeitsprofil und die zweite Position in dem Geschwindigkeitsprofil (P), der ersten Kandidatenposition und der zweiten Kandidatenposition einer Kombination der Vielzahl von Kombinationen, für welche der berechnete Wert der Differenz unter den berechneten Werten der Differenz am größten ist.

7. Das computerimplementierte Verfahren nach einem der Ansprüche 1 bis 6, ferner umfassend eines von:
Bestimmen der ersten Angabe (Z_{B-1}) der ersten Position entlang der axialen Richtung (z) in den OCT-Bildern (10) der ersten Grenze (B-1) der Schicht (L) basierend auf einer Amplitudenkomponente mindestens eines der OCT-Bilder in der Sequenz von OCT-Bildern (10), wobei die zweite Angabe (Z_{B-2}) durch Identifizieren der zweiten Position in dem Geschwindigkeitsprofil (P), die einem durch das Geschwindigkeitsprofil (P) angegebenen Minimum der Geschwindigkeit entspricht, bestimmt wird; und
Bestimmen der zweiten Angabe (Z_{B-2}) der zweiten Position entlang der axialen (z) Richtung in den OCT-Bildern (10) der zweiten Grenze (B-2) der Schicht (L) basierend auf einer Amplitudenkomponente mindestens eines der OCT-Bilder in der Sequenz von OCT-Bildern (10), wobei die erste Angabe (Z_{B-1}) durch das Identifizieren der ersten Position in dem Geschwindigkeitsprofil (P), die einer durch das Geschwindigkeitsprofil (P) angegebenen maximalen Geschwindigkeit entspricht, bestimmt wird.

8. Das computerimplementierte Verfahren nach einem der Ansprüche 1 bis 7, ferner umfassend:
Berechnen eines Vergleichswerts, der eines ist von:
einem Wert einer Bildqualitätsmetrik, der basierend auf mindestens einer einer Amplitudenkomponente des ersten OCT-Bildes (10-1) und einer Amplitudenkomponente des zweiten OCT-Bildes (10-2) berechnet wird; und
einem Wert einer Bildähnlichkeitsmetrik, der ein Maß eines Ähnlichkeitsgrades zwischen Bildern bereitstellt, wobei der Wert der Bildähnlichkeitsmetrik basierend auf der Amplitudenkomponente des ersten OCT-Bildes (10-1) und der Amplitudenkomponente des zweiten OCT-Bildes (10-2) berechnet wird;
Vergleichen des Vergleichswerts mit einem Schwellenwert, um zu bestimmen, ob der Vergleichswert gleich oder größer als der Schwellenwert ist;
in einem Fall, in dem bestimmt wird, dass der Vergleichswert gleich oder größer als der Schwellenwert ist, Erzeugen eines ersten Indikators, der angibt, dass die bestimmte der mindestens einen der ersten Angabe (Z_{B-1}) und der zweiten Angabe (Z_{B-2}) zuverlässig ist; und
in einem Fall, in dem bestimmt wird, dass der Vergleichswert nicht gleich oder größer als der Schwellenwert ist, Erzeugen eines zweiten Indikators, der angibt, dass die bestimmte der mindestens einen der ersten Angabe (Z_{B-1}) und der zweiten Angabe (ZB-2) unzuverlässig ist.

9. Das computerimplementierte Verfahren nach Anspruch 8, wobei der Vergleichswert ein maximaler Wert einer berechneten Kreuzkorrelation zwischen dem ersten OCT-Bild (10-1) und dem zweiten OCT-Bild (10-2) ist.

10. Das computerimplementierte Verfahren nach einem der Ansprüche 1 bis 7, ferner umfassend:
Berechnen, für jeden Satz einer Vielzahl von verschiedenen Sätzen von zwei oder mehr OCT-Bildern (10-1, 10-2) in der Sequenz von OCT-Bildern (10), eines jeweiligen Vergleichswerts, der eines ist von:
einem jeweiligen Wert einer Bildqualitätsmetrik, der basierend auf einer Amplitudenkomponente mindestens eines der OCT-Bilder in dem Satz berechnet wird; und
einem jeweiligen Wert einer Bildähnlichkeitsmetrik, der ein Maß eines Ähnlichkeitsgrades zwischen Bildern bereitstellt, wobei der Wert der Bildqualitätsmetrik basierend auf Amplitudenkomponenten von mindestens zwei der OCT-Bilder in dem Satz berechnet wird;
Vergleichen jedes Vergleichswerts mit einem Schwellenwert, um zu bestimmen, ob der Vergleichswert gleich oder größer als der Schwellenwert ist;
für jeden Satz der Vielzahl von verschiedenen Sätzen von OCT-Bildern, für welchen Satz bestimmt wird, dass der berechnete Vergleichswert gleich oder größer als der Schwellenwert ist, Verarbeiten von Phasenkomponenten der OCT-Bilder in dem Satz, um ein jeweiliges Geschwindigkeitsprofil zu erzeugen, das die Verteilung angibt;
für jeden Satz der Vielzahl von verschiedenen Sätzen von OCT-Bildern, für welchen Satz bestimmt wird, dass der berechnete Vergleichswert nicht größer oder gleich dem Schwellenwert ist, Erzeugen eines jeweiligen Geschwindigkeitsprofils (P), welches die Geschwindigkeit (v) null an allen Positionen entlang der axialen Richtung (z) in dem Geschwindigkeitsprofil (P) angibt;
Erzeugen einer Verkettung (300) der erzeugten Geschwindigkeitsprofile, sodass die Verkettung (300) der Geschwindigkeitsprofile angibt, wie sich die Verteilung über die Zeit ändert; und
Integrieren jeweiliger Abschnitte der Verkettung (300) der Geschwindigkeitsprofile, welche Abschnitte die gleiche Position entlang der axialen Richtung aufweisen, um Daten zu erzeugen, die eine Variation der optischen Weglänge über die Zeit an der Position entlang der axialen Richtung angeben.

11. Das computerimplementierte Verfahren nach Anspruch 10, wobei
die erzeugten Daten einen oder mehrere Sätze von gleichen aufeinanderfolgenden Werten umfassen, und
das Verfahren ferner das Glätten der erzeugten Daten durch das Ersetzen eines oder mehrerer Werte in einem Satz des einen oder der mehreren Sätze von gleichen aufeinanderfolgenden Werten durch einen oder mehrere geschätzte Werte umfasst, die basierend auf benachbarten Werten berechnet werden, die dem Satz von gleichen aufeinanderfolgenden Werten in den erzeugten Daten benachbart sind.

12. Das computerimplementierte Verfahren nach einem der Ansprüche 1 bis 7, ferner umfassend:
Berechnen, für jeden Satz einer Vielzahl von verschiedenen Sätzen von zwei oder mehr OCT-Bildern (10-1, 10-2) in der Sequenz von OCT-Bildern (10), eines jeweiligen Vergleichswerts, der eines ist von:
einem jeweiligen Wert einer Bildqualitätsmetrik, der basierend auf einer Amplitudenkomponente mindestens eines der OCT-Bilder in dem Satz berechnet wird; und
einem jeweiligen Wert einer Bildähnlichkeitsmetrik, der ein Maß eines Ähnlichkeitsgrades zwischen Bildern bereitstellt, wobei der Wert der Bildqualitätsmetrik basierend auf Amplitudenkomponenten von mindestens zwei der OCT-Bilder in dem Satz berechnet wird;
Vergleichen jedes Vergleichswerts mit einem Schwellenwert, um zu bestimmen, ob der Vergleichswert gleich oder größer als der Schwellenwert ist;
für jeden Satz der Vielzahl von verschiedenen Sätzen von OCT-Bildern, für welchen Satz bestimmt wird, dass der berechnete Vergleichswert gleich oder größer als der Schwellenwert ist, Verarbeiten von Phasenkomponenten der OCT-Bilder in dem Satz, um ein jeweiliges Geschwindigkeitsprofil zu erzeugen, das die Verteilung angibt;
für jeden Satz der Vielzahl von verschiedenen Sätzen von OCT-Bildern, für welchen Satz bestimmt wird, dass der berechnete Vergleichswert nicht größer oder gleich dem Schwellenwert ist, Erzeugen eines jeweiligen Geschwindigkeitsprofils (P), welches die Geschwindigkeit (v) null an allen Positionen entlang der axialen Richtung (z) in dem Geschwindigkeitsprofil (P) angibt;
Erzeugen einer Verkettung (300) der erzeugten Geschwindigkeitsprofile, sodass die Verkettung (300) der Geschwindigkeitsprofile angibt, wie sich die Verteilung über die Zeit ändert; und
Integrieren jeweiliger Abschnitte der Verkettung (300) der Geschwindigkeitsprofile, welche Abschnitte die gleiche Position entlang der axialen Richtung aufweisen, um Daten zu erzeugen, die eine Variation der optischen Weglänge über die Zeit an der Position entlang der axialen Richtung angeben.

13. Das computerimplementierte Verfahren nach einem der Ansprüche 10 bis 12, wobei der für jeden Satz berechnete jeweilige Vergleichswert ein jeweiliger maximaler Wert einer berechneten Kreuzkorrelation zwischen mindestens zwei der OCT-Bilder in dem Satz ist.

14. Das computerimplementierte Verfahren nach einem der Ansprüche 10 bis 13, wobei eine jeweilige Angabe einer Variation der optischen Weglänge über die Zeit an jeder von mindestens einer der ersten Position entlang der axialen Richtung (z) und der zweiten Position entlang der axialen Richtung (z), die durch die mindestens eine der ersten Angabe (Z_{B-1}) und der zweiten Angabe (Z_{B-2}) angegeben wird, durch das Integrieren jeweiliger Abschnitte der Verkettung (300) an der mindestens einen der ersten Position entlang der axialen Richtung (z) und der zweiten Position entlang der axialen Richtung (z) bestimmt wird.

15. Das computerimplementierte Verfahren nach einem der Ansprüche 1 bis 14, wobei die Schicht der Retina ein Außensegment von Photorezeptorzellen umfasst.

16. Das computerimplementierte Verfahren nach einem der Ansprüche 1 bis 15, ferner umfassend das Verarbeiten der Phasenkomponente des ersten OCT-Bildes (10-1) und der Phasenkomponente des zweiten OCT-Bildes (10-2), vor der Berechnung des Geschwindigkeitsprofils (P), um eine Gesamtbewegung des gemeinsamen Abschnitts der Retina während der Erfassung der Sequenz von OCT-Bildern (10) durch ein Fourier-Domänen-OCT-Bildgebungssystem (30) nach der Stimulation des gemeinsamen Abschnitts durch den optischen Stimulus (40) zu kompensieren.

17. Ein Computerprogramm (245), umfassend computerlesbare Anweisungen, welche, wenn sie von einem Prozessor (220) ausgeführt werden, den Prozessor (200) veranlassen, ein Verfahren nach einem der Ansprüche 1 bis 16 durchzuführen.

18. Eine Datenverarbeitungsvorrichtung (100), die konfiguriert ist zum Verarbeiten einer Phasenkomponente eines ersten OCT-Bildes (10-1) und einer Phasenkomponente eines zweiten OCT-Bildes (10-2) einer Sequenz von OCT-Bildern (10) eines gemeinsamen Abschnitts einer Retina eines Auges (20), die durch ein Fourier-Domänen-Optische-Kohärenztomographie-, OCT, Bildgebungssystem (30) nach der Stimulation des gemeinsamen Abschnitts durch einen optischen Stimulus (40) erfasst wurde, wobei der gemeinsame Abschnitt eine Schicht (L) der Retina umfasst, deren Dicke sich als Reaktion auf den optischen Stimulus (40) während der Erfassung der Sequenz von OCT-Bildern (10) veränderte, um mindestens eine einer ersten Angabe (ZB-1) einer ersten Position entlang einer axialen Richtung (z) in den OCT-Bildern (10) einer ersten Grenze (B-1) der Schicht (L) und einer zweiten Angabe (ZB-2) einer zweiten Position entlang der axialen Richtung (z) in den OCT-Bildern (10) einer zweiten Grenze (B-2) der Schicht (L) zu bestimmen, wobei die Datenverarbeitungsvorrichtung (100) konfiguriert ist zum:
Verarbeiten (S10) der Phasenkomponente des ersten OCT-Bildes (10-1) und der Phasenkomponente des zweiten OCT-Bildes (10-2), um ein Geschwindigkeitsprofil (P) zu berechnen, das eine Verteilung, entlang der axialen Richtung (z), der Geschwindigkeit (v) innerhalb des gemeinsamen Abschnitts der Retina angibt; und Bestimmen (S20) der mindestens einen der ersten Angabe (Z_{B-1}) und der zweiten Angabe (Z_{B-2}) basierend auf dem berechneten Geschwindigkeitsprofil (P),
wobei die Datenverarbeitungsvorrichtung (100) konfiguriert ist zum Bestimmen der mindestens einen der ersten Angabe (Z_{B-1}) und der zweiten Angabe (Z_{B-2}) basierend auf dem berechneten Geschwindigkeitsprofil durch:
Bestimmen, wenn die erste Angabe (Z_{B-1}) bestimmt ist, einer ersten Position (zₘₐₓ) in dem Geschwindigkeitsprofil (P), die einem durch das Geschwindigkeitsprofil (P) angegebenen Maximum der Geschwindigkeit (vₘₐₓ) entspricht, als die erste Angabe (Z_{B-1}); und
Bestimmen, wenn die zweite Angabe (Z_{B-2}) bestimmt ist, einer zweiten Position (zₘᵢₙ) in dem Geschwindigkeitsprofil (P), die einem durch das Geschwindigkeitsprofil (P) angegebenen Minimum der Geschwindigkeit (vₘᵢₙ) entspricht, als die zweite Angabe (Z_{B-2}).

## Revendications

1. Procédé mis en œuvre par ordinateur consistant à traiter une composante de phase d'une première image OCT (10-1) et une composante de phase d'une deuxième image OCT (10-2) d'une séquence d'images OCT (10) d'une partie commune d'une rétine d'un œil (20), acquises par un système d'imagerie par tomographie en cohérence optique, OCT, de domaine de Fourier (30) après stimulation de la partie commune par un stimulus optique (40), la partie commune comprenant une couche (L) de la rétine dont l'épaisseur a varié en réponse au stimulus optique (40) pendant une acquisition de la séquence d'images OCT (10), pour déterminer au moins une parmi une première indication (Z_{B-1}) d'une première position le long d'une direction axiale (z) dans les images OCT (10) d'une première limite (B-1) de la couche (L) et une deuxième indication (Z_{B-2}) d'une deuxième position le long de la direction axiale (z) dans les images OCT (10) d'une deuxième limite (B-2) de la couche (L), le procédé comprenant :
le traitement (S10) de la composante de phase de la première image OCT (10-1) et de la composante de phase de la deuxième image OCT (10-2) afin de calculer un profil de vitesse (P) indicatif d'une distribution, le long de la direction axiale (z), de la vitesse (v) au sein de la partie commune de la rétine ; et
la détermination (S20) de l'au moins une parmi la première indication (Z_{B-1}) et la deuxième indication (Z_{B-2}) sur la base du profil de vitesse calculé (P), l'au moins une parmi la première indication (Z_{B-1}) et la deuxième indication (Z_{B-2}) étant déterminée sur la base du profil de vitesse calculé (P) en :
déterminant, à l'endroit où la première indication (ZB-1) est déterminée, une première position dans le profil de vitesse (P) correspondant à une maximum de vitesse(v) indiqué par le profil de vitesse (P) en tant que première indication (Z_{B-1}) ; et
déterminant, à l'endroit où la deuxième indication (ZB-2) est déterminée, une deuxième position dans le profil de vitesse (P) correspondant à un minimum de vitesse (v) indiqué par le profil de vitesse (P) en tant que deuxième indication (Z_{B-2}).

2. Procédé mis en œuvre par ordinateur selon la revendication 1, dans lequel l'au moins une parmi la première indication (Z_{B-1}) et la deuxième indication (Z_{B-2}) est déterminée en traitant le profil de vitesse calculé (P) à l'aide d'un algorithme d'analyse par grappes ou d'un algorithme de réduction de dimensionnalité.

3. Procédé mis en œuvre par ordinateur selon la revendication 2, dans lequel l'algorithme de réduction de dimensionnalité comprend un parmi un algorithme d'analyse en composante principale, un algorithme d'analyse en composante indépendante, un algorithme d'analyse discriminante linéaire ou un algorithme de factorisation matricielle non négative.

4. Procédé mis en œuvre par ordinateur selon la revendication 3, dans lequel l'algorithme de réduction de dimensionnalité comprend un algorithme d'analyse en composante principale, et l'au moins une parmi la première indication (Z_{B-1}) et la deuxième indication (Z_{B-2}) est déterminée à l'aide de composantes principales qui ont été déterminées en appliquant l'algorithme d'analyse en composante principale au profil de vitesse calculé (P).

5. Procédé mis en œuvre par ordinateur selon la revendication 1, dans lequel tant la première position dans le profil de vitesse (P) que la deuxième position dans le profil de vitesse (P) sont déterminées en :
calculant, pour chaque combinaison d'une première position candidate dans le profil de vitesse (P) et d'une deuxième position candidate dans le profil de vitesse (P) parmi toutes combinaisons de premières positions candidates et de deuxièmes positions candidates dans le profil de vitesse (P), une valeur respective d'une différence entre une vitesse respective (v) indiquée par le profil de vitesse (P) pour la première position candidate et une vitesse respective (v) indiquée par le profil de vitesse (P) pour la deuxième position candidate de la combinaison ; et
identifiant, comme la première position dans le profil de vitesse (P) et la deuxième position dans le profil de vitesse (P), une première position candidate et une deuxième position candidate d'une combinaison parmi la pluralité de combinaisons pour laquelle la valeur calculée de la différence est la plus grande parmi les valeurs calculées de la différence.

6. Procédé mis en œuvre par ordinateur selon la revendication 1, dans lequel tant la première position dans le profil de vitesse (P) que la deuxième position dans le profil de vitesse (P) sont déterminées en :
calculant, pour chaque combinaison d'une première position candidate dans le profil de vitesse (P) et d'une deuxième position candidate dans le profil de vitesse parmi toutes combinaisons de premières positions candidates et de deuxièmes positions candidates dans le profil de vitesse (P), une valeur respective d'une différence entre une moyenne de vitesses respectives indiquées par le profil de vitesse (P) pour un ensemble de positions adjacentes comprenant la première position candidate de la combinaison, et une moyenne de vitesses respectives indiquées par le profil de vitesse (P) pour un ensemble de positions adjacentes comprenant la deuxième position candidate de la combinaison ; et
identifiant, comme la première position dans le profil de vitesse et la deuxième position dans le profil de vitesse (P), la première position candidate et la deuxième position candidate d'une combinaison parmi la pluralité de combinaisons pour laquelle la valeur calculée de la différence est la plus élevée parmi les valeurs calculées de la différence.

7. Procédé mis en œuvre par ordinateur selon l'une quelconque des revendications 1 à 6, comprenant en outre une des étapes suivantes :
la détermination de la première indication (Z_{B-1}) de la première position le long de la direction axiale (z) dans les images OCT (10) de la première limite (B-1) de la couche (L) sur la base d'une composante d'amplitude d'au moins une des images OCT de la séquence d'images OCT (10), la deuxième indication (Z_{B-2}) étant déterminée en identifiant la deuxième position dans le profil de vitesse (P) correspondant à un minimum de vitesse indiqué par le profil de vitesse (P) ; et
la détermination de la deuxième indication (Z_{B-2}) de la deuxième position le long de la direction axiale (z) dans les images OCT (10) de la deuxième limite (B-2) de la couche (L) sur la base d'une composante d'amplitude d'au moins une des images OCT de la séquence d'images OCT (10), la première indication (Z_{B-1}) étant déterminée en identifiant la première position dans le profil de vitesse (P) correspondant à un maximum de vitesse indiqué par le profil de vitesse (P).

8. Procédé mis en œuvre par ordinateur selon l'une quelconque des revendications 1 à 7, comprenant en outre:
le calcul d'une valeur de comparaison étant une des suivantes :
une valeur d'un indicateur de qualité d'image calculée sur la base d'au moins une parmi une composante d'amplitude de la première image OCT (10-1) et une composante d'amplitude de la deuxième image OCT (10-2) ; et
une valeur d'un indicateur de similarité d'image fournissant une mesure d'un degré de similarité entre images, la valeur de l'indicateur de similarité d'image étant calculée sur la base de la composante d'amplitude de la première image OCT (10-1) et de la composante d'amplitude de la deuxième image OCT (10-2) ;
la comparaison de la valeur de comparaison à un seuil afin de déterminer si la valeur de comparaison est égale ou supérieure au seuil ;
dans un cas où il est déterminé que la valeur de comparaison est égale ou supérieure au seuil, la génération d'un premier indicateur indiquant que l'au moins une déterminée parmi la première indication (Z_{B-1}) et la deuxième indication (Z_{B-2}) sont fiables ; et dans un cas où il est déterminé que la valeur de comparaison n'est pas égale ou supérieure au seuil, la génération d'un deuxième indicateur indiquant que l'au moins une déterminée parmi la première indication (Z_{B-1}) et la deuxième indication (Z_{B-2})ne sont pas fiables.

9. Procédé mis en œuvre par ordinateur selon la revendication 8, dans lequel la valeur de comparaison est une valeur maximale d'une corrélation croisée calculée entre la première image OCT (10-1) et la deuxième image OCT (10-2).

10. Procédé mis en œuvre par ordinateur selon l'une quelconque des revendications 1 à 7, comprenant en outre:
le calcul, pour chaque ensemble d'une pluralité d'ensembles différents de deux images OCT (10-1, 10-2) ou plus dans la séquence d'images OCT (10), d'une valeur de comparaison respective étant une des suivantes :
une valeur respective d'un indicateur de qualité d'image calculé sur la base d'une composante d'amplitude d'au moins une des images OCT de l'ensemble ; et
une valeur respective d'un indicateur de similarité d'image fournissant une mesure d'un degré de similarité entre images, la valeur de l'indicateur de qualité d'image étant calculée sur la base de composantes d'amplitude d'au moins deux des images OCT de l'ensemble ;
la comparaison de chaque valeur de comparaison à un seuil afin de déterminer si la valeur de comparaison est égale ou supérieure au seuil ;
pour chaque ensemble parmi la pluralité d'ensembles différents d'images OCT, pour lequel il est déterminé que la valeur de comparaison calculée est égale ou supérieure au seuil, le traitement de composantes de phase des images OCT de l'ensemble afin de générer un profil de vitesse correspondant qui est indicatif de la distribution ;
pour chaque ensemble parmi la pluralité d'ensembles différents d'images OCT, pour lequel il est déterminé que la valeur de comparaison calculée n'est pas supérieure ou égale au seuil, la génération d'un profil de vitesse respectif (P) qui indique une vitesse (v) nulle en toutes positions le long de la direction axiale (z) dans le profil de vitesse (P) ;
la génération d'une concaténation (300) des profils de vitesse générés de telle sorte que la concaténation (300) des profils de vitesse indique une évolution de la distribution dans le temps ; et
l'intégration de portions respectives de la concaténation (300) des profils de vitesse, lesquelles portions ont la même position le long de la direction axiale, afin de générer des données indiquant une variation de longueur de chemin optique dans le temps à la position le long de la direction axiale.

11. Procédé mis en œuvre par ordinateur selon la revendication 10, dans lequel
les données générées comprennent un ou plusieurs ensembles de valeurs consécutives identiques, et
le procédé comprend en outre le lissage des données générées en remplaçant une ou plusieurs valeurs d'un ensemble parmi le ou les ensembles de valeurs consécutives identiques par une ou plusieurs valeurs estimées, calculées sur la base de valeurs voisines qui sont voisines de l'ensemble de valeurs consécutives identiques dans les données générées.

12. Procédé mis en œuvre par ordinateur selon l'une quelconque des revendications 1 à 7, comprenant en outre:
le calcul, pour chaque ensemble d'une pluralité d'ensembles différents de deux images OCT (10-1, 10-2) ou plus dans la séquence d'images OCT (10), d'une valeur de comparaison respective étant une des suivantes :
une valeur respective d'un indicateur de qualité d'image calculée à partir d'une composante d'amplitude d'au moins une des images OCT de l'ensemble ; et
une valeur respective d'un indicateur de similarité d'image fournissant une mesure du degré de similarité entre images, la valeur de l'indicateur de qualité d'image étant calculée à partir de composantes d'amplitude d'au moins deux des images OCT de l'ensemble ;
la comparaison de chaque valeur de comparaison à un seuil afin de déterminer si la valeur de comparaison est égale ou supérieure au seuil ;
pour chaque ensemble parmi la pluralité d'ensembles différents d'images OCT, pour lequel il est déterminé que la valeur de comparaison calculée est égale ou supérieure au seuil, le traitement de composantes de phase des images OCT de l'ensemble afin de générer un profil de vitesse correspondant qui est indicatif de la distribution ;
pour chaque ensemble parmi la pluralité d'ensembles différents d'images OCT, pour lequel il est déterminé que la valeur de comparaison calculée n'est pas supérieure ou égale au seuil, la génération d'un profil de vitesse correspondant indicatif de la distribution, sur la base d'un profil de vitesse correspondant calculé pour chaque ensemble parmi un ou plusieurs autres ensembles parmi la pluralité d'ensembles différents d'images OCT ;
la génération d'une concaténation (300) des profils de vitesse générés de telle sorte que la concaténation (300) des profils de vitesse indique une évolution de la distribution dans le temps; et
l'intégration de portions respectives de la concaténation (300) des profils de vitesse, lesquelles portions ont la même position le long de la direction axiale, afin de générer des données indiquant une variation de longueur de chemin optique dans le temps à la position le long de la direction axiale.

13. Procédé mis en œuvre par ordinateur selon l'une quelconque des revendications 10 à 12, dans lequel la valeur de comparaison respective calculée pour chaque ensemble est une valeur maximale respective d'une corrélation croisée calculée entre au moins deux des images OCT de l'ensemble.

14. Procédé mis en œuvre par ordinateur selon l'une quelconque des revendications 10 à 13, dans lequel une indication respective d'une variation de longueur de chemin optique dans le temps au niveau de chacune d'au moins une parmi la première position le long de la direction axiale (z) et la deuxième position le long de la direction axiale (z), indiquée par l'au moins une parmi la première indication (Z_{B-1}) et la deuxième indication (Z_{B-2}) est déterminée en intégrant des portions respectives de la concaténation (300) au niveau de l'au moins une parmi la première position le long de la direction axiale (z) et la deuxième position le long de la direction axiale (z).

15. Procédé mis en œuvre par ordinateur selon l'une quelconque des revendications 1 à 14, dans lequel la couche de la rétine comprend un segment externe de cellules photoréceptrices.

16. Procédé mis en œuvre par ordinateur selon l'une quelconque des revendications 1 à 15, comprenant en outre le traitement de la composante de phase de la première image OCT (10-1) et de la composante de phase de la deuxième image OCT (10-2), avant le calcul du profil de vitesse (P), afin de compenser un mouvement global de la partie commune de la rétine lors d'une acquisition de la séquence d'images OCT (10) par un système d'imagerie OCT de domaine de Fourier (30) après stimulation de la partie commune par le stimulus optique (40).

17. Programme informatique (245) comprenant des instructions lisibles par ordinateur qui, lorsqu'elles sont exécutées par un processeur (220), amènent le processeur (200) à mettre en œuvre un procédé selon l'une quelconque des revendications 1 à 16.

18. Dispositif de traitement de données (100) agencé pour traiter une composante de phase d'une première image OCT (10-1) et une composante de phase d'une deuxième image OCT (10-2) d'une séquence d'images OCT (10) d'une partie commune d'une rétine d'un œil (20), acquises par un système d'imagerie par tomographie en cohérence optique, OCT, de domaine de Fourier (30) après stimulation de la partie commune par un stimulus optique (40), dans lequel la partie commune comprend une couche (L) de la rétine dont l'épaisseur a varié en réponse au stimulus optique (40) pendant une acquisition de la séquence d'images OCT (10), afin de déterminer au moins une parmi une première indication (Z_{B-1}) d'une première position le long d'une direction axiale (z) dans les images OCT (10) d'une première limite (B-1) de la couche (L) et une deuxième indication (Z_{B-2}) d'une deuxième position le long de la direction axiale (z) dans les images OCT (10) d'une deuxième limite (B-2) de la couche (L), le dispositif de traitement de données (100) étant agencé pour :
traiter (S10) la composante de phase de la première image OCT (10-1) et la composante de phase de la deuxième image OCT (10-2) afin de calculer un profil de vitesse (P) indiquant une distribution, le long de la direction axiale (z), de vitesse (v) au sein de la partie commune de la rétine ; et
déterminer (S20) l'au moins une parmi la première indication (Z_{B-1}) et la deuxième indication (Z_{B-2}) sur la base du profil de vitesse calculé (P),
le dispositif de traitement de données (100) étant agencé pour déterminer l'au moins une parmi la première indication (Z_{B-1}) et la deuxième indication (Z_{B-2}) sur la base du profil de vitesse calculé en :
déterminant, à l'endroit où la première indication (Z_{B-1}) est déterminée, une première position (zₘₐₓ) dans le profil de vitesse (P) correspondant à un maximum de vitesse (vₘₐₓ) indiqué par le profil de vitesse (P) en tant que première indication (Z_{B-1}) ; et
déterminant, à l'endroit où la deuxième indication (Z_{B-2}) est déterminée, une deuxième position (zₘᵢₙ) dans le profil de vitesse (P) correspondant à une minimum de vitesse (vₘᵢₙ) indiqué par le profil de vitesse (P) en tant que deuxième indication (Z_{B-2}).
